(19) **Europäisches Patentamt / European Patent Office / Office européen des brevets**

(11) **EP 4 137 156 A1**

(12) **EUROPEAN PATENT APPLICATION**
published in accordance with Art. 153(4) EPC

(43) Date of publication:
**22.02.2023 Bulletin 2023/08**

(21) Application number: **21795571.5**

(22) Date of filing: **28.04.2021**

(51) International Patent Classification (IPC):
| | |
|---|---|
| *A61K 39/395* (2006.01) | *A61K 45/00* (2006.01) |
| *A61K 48/00* (2006.01) | *A61P 1/00* (2006.01) |
| *A61P 1/18* (2006.01) | *A61P 11/00* (2006.01) |
| *A61P 35/00* (2006.01) | *A61P 43/00* (2006.01) |
| *C12N 15/113* (2010.01) | *C12Q 1/6886* (2018.01) |
| *G01N 33/15* (2006.01) | *G01N 33/50* (2006.01) |
| *A61K 31/7068* (2006.01) | *A61K 31/7088* (2006.01) |
| *A61K 31/7105* (2006.01) | *A61K 31/713* (2006.01) |

(52) Cooperative Patent Classification (CPC):
**A61K 31/7068; A61K 31/7088; A61K 31/7105;
A61K 31/713; A61K 39/395; A61K 45/00;
A61K 48/00; A61P 1/00; A61P 1/18; A61P 11/00;
A61P 35/00; A61P 43/00; C12N 15/113;
C12Q 1/6886; G01N 33/15;** (Cont.)

(86) International application number:
**PCT/JP2021/017019**

(87) International publication number:
**WO 2021/221116 (04.11.2021 Gazette 2021/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **01.05.2020 JP 2020081351**

(71) Applicants:
• **The University of Tokyo**
  **Bunkyo-ku, Tokyo 113-8654 (JP)**
• **Tak-Circulator Co., Ltd.**
  **Tokyo 113-0033 (JP)**

(72) Inventors:
• **AKIYAMA Tetsu**
  **Tokyo 113-8654 (JP)**
• **HAYASHI Tomoatsu**
  **Tokyo 113-8654 (JP)**
• **YAMAZUMI Yusuke**
  **Tokyo 113-8654 (JP)**
• **ODA Takeaki**
  **Tokyo 113-0033 (JP)**

(74) Representative: **Plasseraud IP**
  **66, rue de la Chaussée d'Antin**
  **75440 Paris Cedex 09 (FR)**

Remarks:
The complete document including Reference Table(s) and the Sequence Listing(s) can be downloaded from the EPO website

(54) **PROPHYLACTIC OR THERAPEUTIC AGENT FOR AT LEAST ONE TYPE OF CANCER SELECTED FROM GROUP CONSISTING OF PANCREATIC CANCER, LUNG CANCER, COLORECTAL CANCER, BILIARY TRACT CANCER AND LIVER CANCER, PROPHYLACTIC OR THERAPEUTIC AGENT FOR SAID CANCER WHICH IS USED IN COMBINATION DRUG IN COMBINATION WITH SAID AGENT, COMBINATION DRUG COMPRISING SAID AGENTS, AND METHOD FOR SCREENING FOR PROPHYLACTIC OR THERAPEUTIC AGENT FOR CANCER**

(57) The present invention provides: a prophylactic or therapeutic agent for at least one type of cancer selected from the group consisting of pancreatic cancer, lung cancer, colorectal cancer, cholangiocarcinoma and liver cancer; a prophylactic or therapeutic agent for the aforementioned cancer, which is used in a combination drug in combination with the aforementioned agent; a combination drug comprising the aforementioned

EP 4 137 156 A1

**(Cont. next page)**

agents; and a method for screening for a prophylactic or therapeutic agent for at least one type of cancer selected from the group consisting of pancreatic cancer, lung cancer, colorectal cancer, cholangiocarcinoma and liver cancer. Provided is a prophylactic or therapeutic agent for at least one type of cancer selected from the group consisting of pancreatic cancer, lung cancer, colorectal cancer, cholangiocarcinoma and liver cancer, the agent comprising a substance capable of reducing the expression of MEX3B gene or MEX3B protein or a substance capable of inhibiting MEX3B protein.

## FIG. 1A

* p-value < 0.05
(Turkey-Kramer test)

## FIG. 1B

* p-value < 0.05
(Turkey-Kramer test)

(52) Cooperative Patent Classification (CPC): (Cont.)
   **G01N 33/50**

**Description**

TECHNICAL FIELD

The present invention relates to: a prophylactic or therapeutic agent for at least one cancer selected from the group consisting of pancreatic cancer, lung cancer, colorectal cancer, cholangiocarcinoma, and liver cancer; a prophylactic or therapeutic agent for at least one cancer selected from the group consisting of pancreatic cancer, lung cancer, colorectal cancer, cholangiocarcinoma, and liver cancer and for use in combination with the above prophylactic or therapeutic agent to form a combination drug; a combination drug comprising the above prophylactic or therapeutic agent; and a method for screening for a prophylactic or therapeutic agent for at least one cancer selected from the group consisting of pancreatic cancer, lung cancer, colorectal cancer, cholangiocarcinoma, and liver cancer.

BACKGROUND ART

In recent years, it has been revealed that cancer cells use immune checkpoint molecules, such as PD-1 and CTLA4, to avoid being attacked by the immune system. Immune checkpoint inhibitors, such as anti-PD-1 and anti-CTLA4 antibodies, have been reported to be significantly effective, in some cases, against malignant melanoma, non-small cell lung cancer, and other cancer types (see, for example, Patent Document 1). Unfortunately, at present, such immune checkpoint inhibitors are effective in only at most 20% of patients who take them. Therefore, a need exists to develop a new anticancer agent that is effective in treating cancer through a different active site, a different action mechanism, or a different route than conventional anticancer agents.

The MEX3B protein, which binds to RNA, is known to function downstream of p53 in the signal cascade pathway. It is also known that the MEX3B protein binds to the 3'UTR (an untranslated region that resides in the exon and does not encode any amino acid) of various target mRNAs to regulate the function of those mRNAs (namely the translation to proteins) or the stability of those mRNAs. For example, Patent Document 2 discloses that the MEX3B protein binds to mRNAs of inflammatory cytokines or chemokines, such as interleukin 6 (IL-6), IL-13, TNF (tumor necrosis factor), G-CSF (granulocyte-colony stimulating factor), CXCL1, CXCL2, and CXCL5, to be involved in the function of those mRNAs (namely the translation to proteins) or the stability of those mRNAs and to be involved in the onset of inflammatory cytokine- or chemokine-induced diseases. The MEX3B protein is also known to be involved in the induction of apoptosis (see, for example, Patent Document 3 and Non-Patent Document 1). Moreover, Non-Patent Document 2 shows that in bronchial asthma model mice, airway inflammation was inhibited when they inhaled an antisense nucleic acid against MEX3B for inhibition of the MEX3B expression in the respiratory tract.

Patent Document 1: Japanese Patent No. 4409430
Patent Document 2: PCT International Publication No. WO2018/008750 A1
Patent Document 3: Japanese Patent No. 4429269

Non-Patent Document 1: Oncogene. 2018 Sep;37(38):5233-5247
Non-Patent Document 2: Cell Rep. 2016 Aug 30;16(9):2456-71.

DISCLOSURE OF THE INVENTION

Problems to be Solved by the Invention

Furthermore, the MEX3B protein has been found to regulate the expression of genes involved in oncogenesis, proliferation, differentiation, and so on, and its expression level has been found to be high in colorectal cancer. The present invention has been made in light of the circumstances described above. It is an object of the present invention to provide: a prophylactic or therapeutic agent for at least one cancer selected from the group consisting of pancreatic cancer, lung cancer, colorectal cancer, cholangiocarcinoma, and liver cancer; a prophylactic or therapeutic agent for at least one cancer selected from the group consisting of pancreatic cancer, lung cancer, colorectal cancer, cholangiocarcinoma, and liver cancer and for use in combination with the above prophylactic or therapeutic agent to form a combination drug; a combination drug comprising the above prophylactic or therapeutic agent; and a method for screening for a prophylactic or therapeutic agent for at least one cancer selected from the group consisting of pancreatic cancer, lung cancer, colorectal cancer, cholangiocarcinoma, and liver cancer.

Means for Solving the Problems

The inventors have completed the present invention based on findings that MEX3B gene knockdown may result in inhibition of growth of at least one cancer selected from the group consisting of pancreatic cancer, lung cancer, colorectal cancer, cholangiocarcinoma, and liver cancer. Specifically, the present invention provides the following aspects.

A first aspect of the present invention is directed to a prophylactic or therapeutic agent for at least one cancer selected from the group consisting of pancreatic cancer, lung cancer, colorectal cancer, cholangiocarcinoma, and liver cancer, the prophylactic or therapeutic agent comprising a substance that reduces expression of an MEX3B gene or an MEX3B protein or comprising a substance inhibiting an MEX3B protein. A second aspect of the present invention is directed to a prophylactic or therapeutic agent for at least one cancer selected from the group consisting of pancreatic cancer, lung cancer, colorectal cancer, cholangiocarcinoma, and liver cancer and for use in combination with the prophylactic or therapeutic agent according to the first aspect to form a combination drug. A third aspect of the present invention is directed to a combination drug (pharmaceutical combination) comprising: the prophylactic or therapeutic agent according to the first aspect; and an additional anticancer agent other than the prophylactic or therapeutic agent. A fourth aspect of the present invention is directed to a method for screening for a prophylactic or therapeutic agent for at least one cancer selected from the group consisting of pancreatic cancer, lung cancer, colorectal cancer, cholangiocarcinoma, and liver cancer, the method comprising screening test substances using at least one indicator selected from the group consisting of a reduction in MEX3B gene expression or MEX3B protein expression and a reduction in MEX3B protein function. The invention may also provide the following aspects. A fifth aspect of the present invention is directed to a method for preventing or treating at least one cancer selected from the group consisting of pancreatic cancer, lung cancer, colorectal cancer, cholangiocarcinoma, and liver cancer, the method comprising administering, to a subject, the prophylactic or therapeutic agent according to the first aspect. A sixth aspect of the present invention is directed to a method for preventing or treating at least one cancer selected from the group consisting of pancreatic cancer, lung cancer, colorectal cancer, cholangiocarcinoma, and liver cancer, the method comprising administering, to a subject, the prophylactic or therapeutic agent according to the first aspect in combination with an additional anticancer agent other than the prophylactic or therapeutic agent.

Effects of the Invention

The prophylactic or therapeutic agent according to the first aspect is useful for preventing or treating at least one cancer selected from the group consisting of pancreatic cancer, lung cancer, colorectal cancer, cholangiocarcinoma, and liver cancer. The prophylactic or therapeutic agent according to the second aspect is useful for preventing or treating at least one cancer selected from the group consisting of pancreatic cancer, lung cancer, colorectal cancer, cholangiocarcinoma, and liver cancer in combination with the prophylactic or therapeutic agent according to the first aspect, which acts through a different active site or a different action mechanism than the agent according to the second aspect. The combination drug according to the third aspect is useful for preventing or treating a certain type of cancer refractory to the additional anticancer agent that acts through a different active site or a different action mechanism than the agent according to the first aspect or is effective in reducing the dosage of the additional anticancer agent. Preferably, the combination drug according to the third aspect allows the prophylactic or therapeutic agent according to the first aspect and the additional anticancer agent to produce a synergetic effect. The method according to the fourth aspect is useful for screening test substances for a prophylactic or therapeutic agent for at least one cancer selected from the group consisting of pancreatic cancer, lung cancer, colorectal cancer, cholangiocarcinoma, and liver cancer.

BRIEF DESCRIPTION OF THE DRAWINGS

FIGS. 1A and 1B are graphs showing results of a pancreatic cancer cell (PK1) growth inhibition test of antisense oligonucleotides against MEX3B;
FIG. 2 is a graph showing a result of a pancreatic cancer cell (AsPC1) growth inhibition test of an antisense oligonucleotide against MEX3B;
FIGS. 3A and 3B are graphs showing results of a non-small cell lung cancer cell growth inhibition test of an antisense oligonucleotide against MEX3B;
FIG. 4 is a graph showing a result of a cholangiocarcinoma cell growth inhibition test of an antisense oligonucleotide against MEX3B;
FIG. 5A is a diagram showing the procedure of a colorectal cancer cell growth inhibition test;
FIGS. 5B, 5C, and 5D are graphs showing results of the colorectal cancer cell growth inhibition test in which an antisense oligonucleotide against MEX3B was administered in combination with an immune checkpoint inhibitor; FIGS. 6A and 6B are graphs showing results of a pancreatic cancer cell growth inhibition test in which an antisense oligonucleotide against MEX3B was administered in combination with a pyrimidine antimetabolite; and

FIG. 7 is a graph showing a result of a liver cancer cell growth inhibition test of an antisense oligonucleotide against MEX3B.

PREFERRED MODE FOR CARRYING OUT THE INVENTION

Hereinafter, embodiments of the present invention will be described in detail, which are not intended to limit the present invention and may be altered or modified as appropriate for implementation without departing from the gist of the present invention.

MEX3B Gene

The MEX3B gene comprises exon 1, an intron, and exon 2, and such a structure is highly conserved among humans, mice, and other mammals. Each of exons 1 and 2 comprises a coding region (CDS) and an untranslated region (UTR). The untranslated regions (UTRs) not encoding amino acids in the exons include 5'UTR upstream of the initiation codon and 3'UTR downstream of the termination codon. The human MEX3B gene encoding the human MEX3B mRNA has the sequence set forth in SEQ ID NO: 1 shown later. In SEQ ID NO: 1, the sequence from nucleotide 437 to 2146 is CDS, the sequence from nucleotide 1 to 436 is 5'UTR, and the sequence from nucleotide 2147 to 3532 is 3'UTR. SEQ ID NO: 2 shown later is a sequence comprising an expression control region located approximately 36 kb upstream of the transcription initiation point of the human MEX3B gene. SEQ ID NO: 3 shown later is the 836-nucleotide sequence of the intronic region in the human MEX3B gene. In the human MEX3B gene, the intronic region is located between nucleotides 694 and 695 of the sequence of SEQ ID NO: 1. SEQ ID NO: 7 is the sequence encoding unspliced human MEX3B pre-mRNA. In the sequence of SEQ ID NO: 7 encoding human MEX3B pre-mRNA, the sequence from nucleotide 437 to 692 and the sequence from nucleotide 1529 to 2982 are each CDS, the sequence from nucleotide 1 to 436 is 5'UTR, the sequence from nucleotide 2983 to 4368 is 3'UTR, and the region from nucleotide 693 to 1528 corresponds to the intronic region represented by SEQ ID NO: 3 in the human MEX3B gene.

The mouse MEX3B gene encoding the mouse MEX3B mRNA has the sequence set forth in SEQ ID NO: 4 shown later. In SEQ ID NO: 4, the sequence from nucleotide 319 to 2049 is CDS, the sequence from nucleotide 1 to 318 is 5'UTR, and the sequence from nucleotide 2050 to 3416 is 3'UTR. Genes encoding MEX3B proteins (e.g., a protein having an amino acid sequence represented by SEQ ID NO: 5 or 6 shown later) all belong to the MEX3B gene. The MEX3B protein encoded by the MEX3B gene is known as a molecule that regulates the function of various mRNAs (namely the translation to proteins) or the stability of those mRNAs by binding to those mRNAs (see, for example, Oncogene. 2018 Sep;37(38):5233-5247). A specific example of the MEX3B gene may be gene (a) or (b) defined below. Gene (a) below is preferred since it is directly available from a human-derived gene without any modification or extra processes, such as transformation.

(a) A gene consisting of the sequence set forth in SEQ ID NO: 1 or 4 in the Sequence Listing.
(b) A gene which consists of the sequence having a deletion, substitution, and/or addition of one or several nucleotides with respect to the sequence set forth in SEQ ID NO: 1 or 4 in the Sequence Listing, and

of which the expression is induced by p53,
which encodes a protein having an activity to induce cellular senescence,
which encodes a protein having an activity to promote the development of at least one cancer selected from the group consisting of pancreatic cancer, lung cancer, colorectal cancer, cholangiocarcinoma, and liver cancer, or
which encodes a protein having an activity to induce the expression of an inflammatory cytokine or chemokine (e.g., IL-6, IL-13, TNF, G-CSF, CXCL1, CXCL2, CXCL5).

The meaning of the term "one or several" as used herein in the expression "having a deletion, substitution, and/or addition of one or several nucleotides with respect to the sequence" is preferably, but not limited to, "1 to 20", more preferably "1 to 10", most preferably "1 to 5". Regarding the DNA mutation level, for example, the mutant DNA typically has a sequence identity of 80% or more, preferably 85% or more, more preferably 90% or more, even more preferably 95% or more, most preferably 98% or more with respect to the MEX3B gene sequence set forth in SEQ ID NO: 1 or 4 in the Sequence Listing.

Acquisition of MEX3B Gene

Any suitable method may be used to acquire the MEX3B gene. Suitable probes or primers may be prepared based on information indicating the nucleotide sequence of SEQ ID NO: 1, 4, or 7 and the amino acid sequence of SEQ ID NO: 5 or 6 in the Sequence Listing provided herein, and then used to select desired clones from a human cDNA library, which is prepared from suitable MEX3B gene-expressing cells by a conventional method, when the MEX3B gene is isolated.

Gene (b) (mutant gene) defined herein above may be prepared by any suitable method known to those skilled in the art, such as chemical synthesis, genetic engineering, or mutagenesis. For example, mutant DNA may be obtained from the DNA with the sequence of SEQ ID NO: 1 by introducing a mutation(s) into the DNA. Specifically, mutant DNA may be obtained by a method of bringing a mutagenic agent into contact with the DNA with the sequence of SEQ ID NO: 1 or 4 to allow the agent to act on the DNA, a method of exposing the DNA to ultraviolet light, or a genetic engineering method.

MEX3B Protein

The MEX3B protein may be one of the following proteins:

(a) a protein consisting of the amino acid sequence set forth in SEQ ID NO: 5 or 6 in the Sequence Listing; and
(b) a protein which consists of the sequence having a deletion, substitution, and/or addition of one or several amino acids with respect to the amino acid sequence set forth in SEQ ID NO: 5 or 6 in the Sequence Listing or has an amino acid sequence identity of 95% or more with respect to the sequence set forth in SEQ ID NO: 5 or 6 in the Sequence Listing, and

of which the expression is induced by p53,
which has an activity to induce cellular senescence, or
which has an activity to promote development of at least one cancer selected from the group consisting of pancreatic cancer, lung cancer, colorectal cancer, cholangiocarcinoma, and liver cancer or to induce expression of an inflammatory cytokine or chemokine (e.g., IL-6, IL-13, TNF, G-CSF, CXCL1, CXCL2, CXCL5).

Protein (a) defined above is preferred since it is directly available from a human-derived protein without any modification or extra processes, such as transformation. SEQ ID NO: 5 is the amino acid sequence of the human MEX3B protein. SEQ ID NO: 6 is the amino acid sequence of the mouse MEX3B protein.

The meaning of the term "one or several" as used herein in the expression "having a deletion, substitution, and/or addition of one or several amino acids with respect to the amino acid sequence" is preferably, but not limited to, "1 to 10", more preferably "1 to 5", most preferably "1 to 3". As used herein, the term "an amino acid sequence identity of 95% or more" may mean an amino acid sequence homology of 95% or more. The protein preferably has an amino acid sequence identity of 96% or more, more preferably 970 or more. Physiologically active proteins encoded by mutant genes having a high sequence identity with respect to the gene with the sequence of SEQ ID NO: 1 or 4 in the Sequence Listing as stated above and having the ability to bind to a specific mRNA will all fall within the scope of the present invention. Different amino acid side chains for serving as protein components have different hydrophobic properties, electric charges, sizes, or other properties. It is, however, known empirically or from physicochemical measurements that some amino acids can be substituted for other amino acids without substantially affecting the three-dimensional structure (also referred to as "conformation") of a protein as a whole, in other words, in a highly conservative way. Examples of such conservative substitutions of amino acid residues include those between glycine (Gly) and proline (Pro), Gly and alanine (Ala) or valine (Val), leucine (Leu) and isoleucine (Ile), glutamic acid (Glu) and glutamine (Gln), aspartic acid (Asp) and asparagine (Asn), cysteine (Cys) and threonine (Thr), Thr and serine (Ser) or Ala, and lysine (Lys) and arginine (Arg).

Therefore, physiologically active mutant proteins consisting of the sequence having a deletion, substitution, and/or addition of one or more amino acids with respect to the MEX3B amino acid sequence of SEQ ID NO: 5 or 6 in the Sequence Listing, having the ability to bind to a specific mRNA like the MEX3B protein, and being mutated in a highly conservative manner with respect to the three-dimensional structure of the MEX3B protein will all fall within the range of MEX3B proteins. MEX3B proteins may be acquired by any suitable method. MEX3B proteins may be synthetic proteins, such as those chemically synthesized, naturally-occurring proteins isolated from biological samples, cultured cells, or other sources, or recombinant proteins produced by genetic recombination technology.

Prophylactic or Therapeutic Agent for at Least One Cancer Selected from the Group Consisting of Pancreatic Cancer, Lung Cancer, Colorectal Cancer, Cholangiocarcinoma, and Liver Cancer

The prophylactic or therapeutic agent according to the first aspect for at least one cancer selected from the group consisting of pancreatic cancer, lung cancer, colorectal cancer, cholangiocarcinoma, and liver cancer (hereinafter also simply referred to as "the prophylactic or therapeutic agent according to the first aspect") comprises, as an active component, a substance that reduces expression of an MEX3B gene or an MEX3B protein or comprises a substance inhibiting an MEX3B protein as an active component. The prophylactic or therapeutic agent according to the first aspect preferably comprises, as an active component, a substance that reduces expression of an MEX3B gene or an MEX3B protein, and more preferably comprises, as an active component, a substance that reduces expression of an MEX3B gene.

The substance that reduces expression of an MEX3B gene or an MEX3B protein may be an antisense oligonucleotide, a nucleic acid having RNAi activity (e.g., siRNA, shRNA), miRNA, an artificial nuclease, or a low-molecular-weight compound, which will be described below. The substance inhibiting an MEX3B protein may be any substance that inhibits the function of the MEX3B protein. Specifically, the substance inhibiting an MEX3B protein may be a polymer compound (e.g., a nucleic acid, such as an aptamer), an antibody, or a low-molecular-weight compound, which will be described later.

Antisense Oligonucleotide

The substance that reduces expression of an MEX3B gene or an MEX3B protein may be an antisense oligonucleotide having a sequence complementary to a contiguous sequence in the MEX3B gene (in the CDS or UTR of the exon or in the intron) or in the expression control region of the MEX3B gene. The substance that reduces expression of an MEX3B gene or an MEX3B protein is preferably an antisense oligonucleotide having a sequence complementary to an oligonucleotide in the MEX3B gene (in the CDS or UTR of the exon) or in the expression control region of the MEX3B gene, more preferably an antisense oligonucleotide having a sequence complementary to an oligonucleotide in the MEX3B gene (in the CDS or UTR of the exon), even more preferably an antisense oligonucleotide having a sequence complementary to an oligonucleotide in the UTR of the MEX3B gene, furthermore preferably an antisense oligonucleotide having a sequence complementary to an oligonucleotide in the 3'UTR of the MEX3B gene, most preferably an antisense oligonucleotide having a sequence complementary to an oligonucleotide in the sequence from nucleotide 3129 to 4293 in the sequence of SEQ ID NO: 7 encoding human MEX3B pre-mRNA. When introduced into cells (preferably into nuclei), the antisense oligonucleotide will inhibit the transcription or translation of the MEX3B gene and act effectively in preventing or treating at least one cancer selected from the group consisting of pancreatic cancer, lung cancer, colorectal cancer, cholangiocarcinoma, and liver cancer. For example, when introduced into cells (preferably into nuclei), the antisense oligonucleotide will hybridize to the complementary oligonucleotide in the MEX3B gene (in the CDS or UTR of the exon or in the intron) or in the expression control region of the MEX3B gene, and the resulting hybrid duplex (nucleotide chain-containing MEX3B mRNA) will be degraded by a nuclease (e.g., RNase H) specific for the hybrid duplex, which will result in the inhibition of the transcription or translation of the MEX3B gene. The antisense oligonucleotide may be DNA or RNA. Preferably, the antisense oligonucleotide is DNA for the mRNA cleavage by the specific nuclease. The antisense oligonucleotide may have a sequence complementary to any number of contiguous nucleotides that are necessary for the inhibition of the expression of the MEX3B gene and present in the sequence of the MEX3B gene (in the CDS or UTR of the exon or in the intron) or in the expression control region of the MEX3B gene. Typically, the antisense oligonucleotide preferably has a sequence complementary to an oligonucleotide comprising at least 10 contiguous nucleotides, more preferably has a sequence complementary to an oligonucleotide comprising at least 11 contiguous nucleotides, even more preferably has a sequence complementary to an oligonucleotide comprising at least 12 contiguous nucleotides, furthermore preferably has a sequence complementary to an oligonucleotide comprising at least 13 contiguous nucleotides, most preferably has a sequence complementary to an oligonucleotide comprising at least 14 contiguous nucleotides, in the sequence of the MEX3B gene (in the CDS or UTR of the exon or in the intron) or in the expression control region of the MEX3B gene. Regarding the upper limit of the length of the antisense oligonucleotide, the antisense oligonucleotide preferably has a sequence complementary to an oligonucleotide comprising at most 40 contiguous nucleotides, more preferably has a sequence complementary to an oligonucleotide comprising at most 30 contiguous nucleotides, even more preferably has a sequence complementary to an oligonucleotide comprising at most 25 contiguous nucleotides, furthermore preferably has a sequence complementary to an oligonucleotide comprising at most 20 contiguous nucleotides, still more preferably has a sequence complementary to an oligonucleotide comprising at most 17 contiguous nucleotides, most preferably has a sequence complementary to an oligonucleotide comprising at most 16 contiguous nucleotides, in the sequence of the MEX3B gene (in the CDS or UTR of the exon or in the intron) or in the expression control region of the MEX3B gene.

The antisense oligonucleotide may or may not be an artificially synthesized nucleic acid. The antisense oligonucleotide preferably contains at least one nucleotide having at least one structure selected from the group consisting of a phosphorothioate structure, a cross-linked structure, and an alkoxy structure. For example, when having a phosphorothioate bond in place of a phosphodiester bond between nucleotides, the antisense oligonucleotide will be nuclease-resistant and have improved hydrophobicity, which will improve its uptake into cells or nuclei. The antisense oligonucleotide will also be nuclease-resistant and have an improved ability to bind to mRNA when containing a nucleotide having a cross-linked structure at the sugar moiety, such as 2',4'-BNA (2',4'-bridged nucleic acid also called LNA (locked nucleic acid)) or ENA (2'-O,4'-C-ethylene-bridged nucleic acid) or containing a nucleotide having an alkoxy structure, such as a 2'-O-methyl or 2'-O-methoxyethyl (2'-MOE) moiety. The antisense oligonucleotide preferably has at least one phosphorothioate bond in place of a phosphodiester bond between nucleotides. The antisense oligonucleotide more preferably has phosphorothioate bonds in place of at least 50% of the phosphodiester bonds, even more preferably in place of at least 70% of the phosphodiester bonds, furthermore preferably in place of at least 90% of the phosphodiester bonds, most preferably in place of all of the phosphodiester bonds. The antisense oligonucleotide preferably has at least one nucleotide modified to have a cross-linked structure or an alkoxy structure on at least one of the terminal sides (preferably one to three nucleotides modified to have a cross-linked structure or an alkoxy structure on the terminal side). The antisense oligonucleotide more preferably has nucleotides modified to have a cross-liked structure or an alkoxy structure on each of the two terminal sides (what is called a gapmer-type antisense oligonucleotide), even more preferably has nucleotides modified independently to have a cross-linked structure or an alkoxy structure from the end to the fourth nucleotide on each of the two terminal sides, and most preferably has nucleotides modified independently to have a cross-linked structure or an alkoxy structure from the end to the second or third nucleotide on each of the two terminal sides. In the antisense oligonucleotide, cytosine (cytidine) at any site may or may not be modified by methylation at position 5. The antisense oligonucleotide may be produced by a conventional method using a DNA synthesizer and known organic synthesis techniques.

The antisense oligonucleotide may be delivered into cells (preferably into nuclei) by free uptake. The prophylactic or therapeutic agent according to the first aspect may contain any transfection agent, which will improve cellular uptake, or may not contain it. The transfection agent may include polyethyleneimine (PEI), and preferably includes linear PEI. Linear PEI can be synthesized by hydrolysis of poly(2-ethyl-2-oxazoline). The transfection agent including linear PEI is commercially available under the name jetPEI (registered trademark, manufactured by Polyplus-transfection). The commercially available transfection agent, in vivo-jetPEI (registered trademark), is preferred. For example, the prophylactic or therapeutic agent according to the first aspect may contain the transfection agent in an amount that provides an N/P ratio (the number of PEI nitrogen residues per phosphate of nucleic acid) of 1 to 30, preferably 1 to 10, more preferably 2 to 5.

The prophylactic or therapeutic agent according to the first aspect may further comprise any drug delivery system (DDS) or DDS agent, which will improve cellular uptake, or may not contain that. The antisense oligonucleotide may or may not be incorporated in any drug delivery system (DDS) or DDS agent. The DDS agent may comprise, for example, particles with a size of 300 nm or less, preferably 200 nm or less, more preferably 100 nm or less. Such particles are preferably monodisperse particles having a core-shell structure, which is preferably formed by self-assembly.

Such particles preferably comprise polymeric micelles. The polymeric micelles may comprise a block copolymer comprising polyethylene glycol (PEG) and poly(amino acid). The block copolymer and the antisense oligonucleotide are preferably assembled into polymeric micelles. The DDS agent preferably comprises a ligand molecule capable of binding to the target (preferably target cells). More preferably, the DDS agent comprises polymeric micelles comprising PEG and the ligand molecule bonded to the PEG (preferably bonded to the top of the PEG). The ligand molecule may be a molecule capable of targeting various cancer cells, such as cyclic RGD (cRGD) peptide comprising arginine, glycine, and aspartic acid, an antibody fragment, lactose, folic acid, or phenylboronic acid, among which cRGD peptide is preferred. The DDS agent may be one of those shown in Miyata K. et al. React. Funct. Polym. 71, 227-234 (2011) and Miyata K. Drug Discov. Ther. 10, 236-247 (2016).

The prophylactic or therapeutic agent according to the first aspect may further comprise a lipofection vehicle, which will improve cellular uptake, or may not comprise it. The lipofection vehicle may be a vehicle having a high affinity for cell membranes (e.g., liposomes, cholesterols). The lipofection vehicle is preferably Lipofectamine or Lipofectin, more preferably Lipofectamine.

For example, the antisense oligonucleotide may be administered alone or in combination with the transfection agent, the DDS agent, or the lipofection vehicle to a subject (e.g., a patient, an asymptomatic patient) locally or systemically by injection or other means (e.g., intratumorally, intravenously, intraperitoneally, locally transdermally, by inhalation) for prevention or treatment of at least one cancer selected from the group consisting of pancreatic cancer, lung cancer, colorectal cancer, cholangiocarcinoma, and liver cancer. The uptake of the antisense oligonucleotide into cells or nuclei of the subject (e.g., patient, asymptomatic patient) will be improved when the antisense oligonucleotide has at least one structure selected from the group consisting of a phosphorothioate structure, a cross-linked structure, and an alkoxy structure and is used in combination with the lipofection vehicle. The dosage of the antisense oligonucleotide used as an active component may be usually in the range of about 0.1 $\mu$g to about 100 mg per kg body weight per dose.

In an embodiment of the present invention, the delivery of the antisense oligonucleotide into cells (preferably nuclei) may be achieved by infecting target cancer cells with a vector or virus prepared by a process that includes inserting the antisense oligonucleotide into a suitable vector or virus; and introducing the product into suitable packaging cells to produce the vector or virus. The suitable vector or virus may be any type, such as an autonomously replicating vector or virus. Preferably, the suitable vector or virus is such that when introduced into a packaging cell, it will be incorporated into the genome of the packaging cell and replicated with the chromosome having it incorporated therein.

If necessary, the antisense oligonucleotide may be operably linked to a suitable terminator, such as a human growth hormone terminator or a TPI1 or ADH3 terminator for fungal hosts. The recombinant vector may contain additional elements, such as a polyadenylation signal (e.g., one derived from SV40 or adenoviral 5E1b region), a transcriptional enhancer sequence (e.g., SV40 enhancer), and a translational enhancer sequence (e.g., one encoding adenovirus VA RNA). The recombinant vector may further comprise a DNA sequence that allows the vector or virus to replicate in the packaging cell, an example of which is an SV40 replication origin.

The packaging cells, into which the antisense oligonucleotide or a vector or virus containing the antisense oligonucleotide is to be introduced for the production of the vector or virus, may be higher eukaryotic cells, bacterial cells, yeast cells, fungal cells, or other cells, among which mammalian cells are preferred. Examples of mammalian cells include HEK293 cells (e.g., HEK293FT cells, HEK293T cells). A known method, such as lipofection, electroporation, or calcium phosphate method, may be used to transform mammalian cells with the gene to be expressed.

Nucleic Acid Having RNAi Activity

A nucleic acid having RNAi activity, which will inhibit expression of an MEX3B gene, is a preferred example of the substance that reduces expression of an MEX3B gene or an MEX3B protein. The nucleic acid having RNAi activity preferably comprises a partial contiguous sequence of the coding or untranslated region of an RNA sequence transcribed from the MEX3B gene or preferably comprises a sequence complementary to the partial contiguous sequence. RNAi (RNA interference) refers to the phenomenon in which the expression of a certain target gene is suppressed by introducing, into cells, double-stranded RNA (dsRNA) comprising part of mRNA encoding part of the target gene. The nucleic acid having RNAi activity may be siRNA (small interfering RNA) or shRNA (small hairpin RNA). Hereinafter, siRNA and shRNA will be described.

(1) siRNA

The siRNA may be a double-stranded RNA having RNAi activity to inhibit the expression of the MEX3B gene. A DNA encoding such a double-stranded RNA may also be used. The siRNA is preferably a double-stranded RNA having RNAi activity to inhibit the expression of the MEX3B gene and having a partial contiguous sequence of an RNA sequence transcribed from the MEX3B gene. A DNA encoding such a double-stranded RNA is also preferably used. More specifically, the siRNA may have a sequence complementary to any number of contiguous nucleotides that are necessary for the inhibition of the expression of the MEX3B gene and present in the CDS or UTR of an RNA sequence transcribed from the MEX3B gene. Typically, the siRNA is preferably a double-stranded RNA comprising at least 17 contiguous nucleotides. A DNA encoding such a double-stranded RNA is also preferably used. More preferably, the siRNA is a double-stranded RNA comprising at least 17 contiguous nucleotides of the UTR of an RNA sequence transcribed from the MEX3B gene. A DNA encoding such a double-stranded RNA is also more preferably used. Even more preferably, the siRNA is a double-stranded RNA comprising at least 17 contiguous nucleotides of the 3'UTR of an RNA sequence transcribed from the MEX3B gene. A DNA encoding such a double-stranded RNA is also even more preferably used. Most preferably, the siRNA is a double-stranded RNA comprising at least 17 contiguous nucleotides of an RNA sequence transcribed from the sequence from nucleotide 3129 to 4293 in the sequence of SEQ ID NO: 7 encoding human MEX3B pre-mRNA. A DNA encoding such a double-stranded RNA is also most preferably used.

The siRNA may have a sequence complementary to any number of contiguous nucleotides that are necessary for the inhibition of the expression of the MEX3B gene and present in the CDS or UTR of an RNA sequence transcribed from the MEX3B gene. Typically, the siRNA is preferably a double-stranded RNA comprising at least 18 contiguous nucleotides. A DNA encoding such a double-stranded RNA is also preferably used. More preferably, the siRNA is a double-stranded RNA comprising at least 19 contiguous nucleotides of the CDS or UTR of an RNA sequence transcribed from the MEX3B gene. A DNA encoding such a double-stranded RNA is also more preferably used. Even more preferably, the siRNA is a double-stranded RNA comprising at least 20 contiguous nucleotides of the CDS or UTR of an RNA sequence transcribed from the MEX3B gene. A DNA encoding such a double-stranded RNA is also even more preferably used. Most preferably, the siRNA is a double-stranded RNA comprising at least 21 contiguous nucleotides of the CDS or UTR of an RNA sequence transcribed from the MEX3B gene. A DNA encoding such a double-stranded RNA is also most preferably used. The siRNA is preferably a double-stranded RNA comprising at most 30 contiguous nucleotides of the CDS or UTR of an RNA sequence transcribed from the MEX3B gene. A DNA encoding such a double-stranded RNA is also preferably used. More preferably, the siRNA is a double-stranded RNA comprising at most 25 contiguous nucleotides of the CDS or UTR of an RNA sequence transcribed from the MEX3B gene. A DNA encoding such a double-stranded RNA is also more preferably used.

The DNA encoding the double-stranded RNA may be, for example, a DNA having an inverted repeat sequence corresponding to a partial sequence of the MEX3B gene. A DNA having such an inverted repeat sequence may be introduced into mammalian cells to intracellularly express an inverted repeat sequence corresponding to a partial sequence of the MEX3B gene, which will have RNAi activity to inhibit the expression of the target gene (MEX3B). The term "inverted repeat sequence" refers to a sequence comprising: a partial sequence of the target gene (MEX3B); another sequence complementary and opposite in direction to the partial sequence; and a suitable sequence intervening between those sequences, in which those sequences are arranged in parallel with the suitable sequence intervening them. Specifically, assuming that a partial sequence of the target gene is a double strand consisting of n nucleotide pairs as shown below

$$5'-X_1X_2 \ \ldots \ X_{n-1}X_n-3'$$

$$3'-Y_1Y_2 \ \ldots \ Y_{n-1}Y_n-5',$$

the corresponding inverted sequence is as follows:

$$5'-Y_nY_{n-1} \ \ldots \ Y_2Y_1-3'$$

$$3'-X_nX_{n-1} \ \ldots \ X_2X_1-5'$$

in which nucleotide X is complementary to nucleotide Y when they have the same numerical subscript.

The inverted repeat sequence comprises two sequences, such as those shown above, and a suitable sequence intervening between them. In the inverted repeat sequence, a partial sequence of the target gene may be located upstream or downstream of its reverse complement. Preferably, in the inverted repeat sequence used in the present invention, a partial sequence of the target gene is located downstream of its reverse complement.

(2) shRNA

The shRNA may be a single-stranded RNA having an inverted repeat sequence comprising: a partial sequence of an RNA sequence transcribed from the MEX3B gene; its reverse complement; and an intervening sequence capable of forming a hairpin loop between the partial sequence and its reverse complement, which are arranged in parallel. A DNA encoding such a single-stranded RNA may also be used. A method of introducing an shRNA expression vector or virus into cells is advantageously used. Similar to the siRNA, the shRNA will function in cells.

In the shRNA, the partial sequence of an RNA sequence transcribed from the MEX3B gene may be complementary to any number of contiguous nucleotides that are necessary for the inhibition of the expression of the MEX3B gene and present in the CDS or UTR of an RNA sequence transcribed from the MEX3B gene. Typically, in the shRNA, the partial sequence preferably comprises at least 17 contiguous nucleotides, more preferably comprises at least 17 contiguous nucleotides of the UTR of an RNA sequence transcribed from the MEX3B gene, even more preferably comprises at least 17 contiguous nucleotides of the 3'UTR of an RNA sequence transcribed from the MEX3B gene, most preferably comprises at least 17 contiguous nucleotides of an RNA sequence transcribed from the sequence from nucleotide 3129 to 4293 in the sequence of SEQ ID NO: 7 encoding human MEX3B pre-mRNA.

The partial sequence of an RNA sequence transcribed from the MEX3B gene preferably comprises at least 18 contiguous nucleotides of the CDS or UTR of the RNA sequence transcribed from the MEX3B gene, more preferably comprises at least 19 contiguous nucleotides of the CDS or UTR of the RNA sequence transcribed from the MEX3B gene, even more preferably comprises at least 20 contiguous nucleotides of the CDS or UTR of the RNA sequence transcribed from the MEX3B gene, most preferably comprises at least 21 contiguous nucleotides of the CDS or UTR of the RNA sequence transcribed from the MEX3B gene. The partial sequence of an RNA sequence transcribed from the MEX3B gene preferably comprises at most 30 contiguous nucleotides of the CDS or UTR of the RNA sequence transcribed from the MEX3B gene, more preferably comprises at most 25 contiguous nucleotides of the CDS or UTR of the RNA sequence transcribed from the MEX3B gene.

The sequence capable of forming a hairpin loop may have any length that allows the formation of a hairpin loop. The sequence capable of forming a hairpin loop is preferably 0 to 300 bp long, more preferably 1 to 100 bp long, even more preferably 2 to 75 bp long, most preferably 3 to 50 bp long. This sequence may have a restriction enzyme site.

The inverted repeat sequence of the target gene may be incorporated downstream of a promoter operable in mammalian cells so that it can be expressed in the mammalian cells. The promoter sequence may be any type operable in mammalian cells.

miRNA

A miRNA (micro RNA), which will inhibit the expression of the MEX3B gene, is also a preferred example of the substance that reduces expression of an MEX3B gene or an MEX3B protein. The miRNA can pair with the 3'UTR of mRNA to inhibit the translation of the MEX3B gene. More specifically, the miRNA is transcribed as an RNA precursor with a hairpin-like structure, which will be cleaved by dsRNA cleavage enzyme with RNase III cleavage activity and then incorporated into RISC or RISC-like protein complexes to inhibit the translation of the mRNA. In the present invention, the miRNA is intended to include all of pri-miRNA (primary miRNA), pre-miRNA, and mature miRNA. In the present invention, the miRNA preferably comprises a partial contiguous sequence of the 3'UTR of an RNA transcribed from the MEX3B gene or preferably comprises a sequence complementary to the partial contiguous sequence. More preferably, the miRNA comprises a partial sequence of an RNA sequence transcribed from the sequence from nucleotide 3129 to 4293 in the sequence of SEQ ID NO: 7 encoding human MEX3B pre-mRNA or comprises a sequence complementary to the partial sequence. The partial sequence may have any length. The partial sequence is preferably 7 nucleotides or more in length, more preferably 8 nucleotides or more in length, even more preferably 9 nucleotides or more in length, furthermore preferably 11 nucleotides or more in length, still more preferably 13 nucleotides or more in length, yet more preferably 15 nucleotides or more in length, most preferably 17 nucleotides or more in length. The upper limit length of the partial sequence is preferably, but not limited to, 50 nucleotides or less, more preferably 40 nucleotides or less, even more preferably 30 nucleotides or less, furthermore preferably 25 nucleotides or less, most preferably 23 nucleotides or less. The pri-miRNA is commonly several hundred to several thousand nucleotides in length, and the pre-miRNA is commonly 50 to 80 nucleotides in length.

The nucleic acid having RNAi activity or the miRNA may be delivered into cells (preferably into nuclei) by free uptake. In an embodiment of the present invention, the delivery of the nucleic acid having RNAi activity or the miRNA into cells may be achieved by infecting target cancer cells with a vector or virus prepared by a process that includes inserting the nucleic acid or the miRNA into a suitable vector or virus; and introducing the product into suitable packaging cells to produce the vector or virus. The suitable vector or virus may be any type, such as an autonomously replicating vector or virus. Preferably, the suitable vector or virus is such that after being introduced into the packaging cell, it will be incorporated into the genome of the packaging cell and replicated with the chromosome having it incorporated therein. Examples of the suitable vector or virus include plasmids derived from E. coli (e.g., pBR322, pUC118), plasmids derived from B. subtilis (e.g., pUB110, pSH19), and animal viruses, such as lentiviruses, retroviruses, adenoviruses, bacteriophages, and vaccinia viruses. The recombination may be performed using suitable synthetic DNA adapters to add a translation initiation codon and a translation termination codon.

If necessary, the nucleic acid having RNAi activity or the miRNA may be operably linked to a suitable terminator, such as a human growth hormone terminator or a TPI1 or ADH3 terminator for fungal hosts. The recombinant vector may contain additional elements, such as a polyadenylation signal (e.g., one derived from SV40 or adenoviral 5E1b region), a transcriptional enhancer sequence (e.g., SV40 enhancer), and a translational enhancer sequence (e.g., one encoding adenovirus VA RNA). The recombinant vector or virus may further comprise a DNA sequence that allows the vector or virus to replicate in the packaging cell, an example of which is an SV40 replication origin. The recombinant vector or virus may further contain a selection marker. The selection marker may be, for example, a gene for which the complement is missing in the packaging cell, such as a dihydrofolate reductase (DHFR) gene or a Schizosaccharomyces pombe TPI gene, or a drug resistance gene, such as a gene resistant to ampicillin, kanamycin, tetracycline, chloramphenicol, neomycin, or hygromycin.

The packaging cells, which are used to produce a vector or virus having the nucleic acid with RNAi activity or the miRNA incorporated therein, may be higher eukaryotic cells, bacterial cells, yeast cells, fungal cells, or other cells, among which mammalian cells are preferred. Examples of mammalian cells include HEK293 cells (e.g., HEK293FT cells, HEK293T cells). A known method, such as lipofection, electroporation, or calcium phosphate method, may be used to transform mammalian cells with the gene to be expressed.

Similar to the antisense oligonucleotide, the nucleic acid having RNAi activity or the miRNA may be used in combination with the transfection agent, the lipofection vehicle, or the DDS agent described above.

For example, the nucleic acid having RNAi activity or the miRNA may be administered alone or in combination with the transfection agent, the DDS agent, or the lipofection vehicle (used as an aid for cellular uptake) to a subject (e.g., a patient, an asymptomatic patient) locally or systemically by injection or other means (e.g., intratumorally, intravenously, intraperitoneally, locally transdermally, by inhalation) so that it can be delivered into cells of the subject (e.g., patient, asymptomatic patient). The dosage of the double-stranded RNA or DNA used as an active component may be usually in the range of about 0.1 μg to about 10 mg per kg body weight per dose.

Artificial Nuclease

The substance that reduces expression of an MEX3B gene or an MEX3B protein may also be an artificial nuclease for genome editing, such as CRISPR (clustered regularly interspaced short palindromic repeats)-Cas nuclease, or an artificial restriction enzyme (artificial nuclease), such as a TALEN (transcription activator-like effector nuclease) or a ZFN (zinc finger nuclease). A TALEN is an artificial nuclease comprising TALEs and a DNA cleavage domain, in which the TALEs are domains resulting from linkage of four units capable of recognizing and biding to four nucleotides (A, T, G, and C), respectively. In such a TALEN, the TALEs recognize and bind to at least a partial sequence of the MEX3B gene. A ZFN is an artificial nuclease in the form of a chimeric protein comprising a zinc finger domain and a DNA cleavage domain. The zinc finger domain comprises multiple zinc finger motifs linked together, each of which recognizes a specific threenucleotide sequence. The zinc finger domain recognizes and binds to a DNA sequence of nucleotides in multiples of 3. In such a ZFN, the zing finger domain recognizes and binds to at least a partial sequence of the MEX3B gene.

A CRISPR-Cas nuclease comprises a guide RNA and a Cas nuclease (preferably Cas9). The guide RNA is an RNA that binds to the Cas nuclease, which is a DNA cleavage enzyme, and functions to guide the Cas nuclease to the target DNA (at least a partial sequence of the MEX3B gene). The guide RNA has, at its 5' end, a sequence complementary to the target DNA (at least a partial sequence of the MEX3B gene) and utilizes the complementary sequence to bind to the target DNA and to guide the Cas nuclease to the target DNA. The Cas nuclease functions as a DNA endonuclease to cleave DNA at the site of the target DNA, for example, which will lead to a specific reduction in the expression of the MEX3B gene. At least a partial sequence to be targeted in the MEX3B gene may be an oligonucleotide in the MEX3B gene (in the CDS or URT of the exon or in the intron) or in the expression control region of the MEX3B gene. To ensure a reduction in the expression of the MEX3B gene, at least a partial sequence targeted in the MEX3B gene is preferably an oligonucleotide in the MEX3B gene (in the CDS or UTR of the exon) or in the expression control region of the MEX3B gene, more preferably an oligonucleotide in the MEX3B gene (in the CDS of the exon) or in the expression control region of the MEX3B gene, even more preferably an oligonucleotide in the MEX3B gene (in the CDS of the exon), furthermore preferably an oligonucleotide in the MEX3B gene (in the CDS of exon 1), most preferably an oligonucleotide comprising the initiation codon of the MEX3B gene. The partial sequence to be targeted in the MEX3B gene is preferably 15 to 25 nucleotides in length, more preferably 17 to 22 nucleotides in length, even more preferably 18 to 21 nucleotides in length, most preferably 20 nucleotides in length.

There may be provided a composition comprising: a guide RNA specific for the MEX3B gene or a DNA encoding the guide RNA; and a Cas nuclease or a nucleic acid encoding the Cas nuclease. Eukaryotic cells or organisms having the MEX3B gene may be transfected with such a composition for a reduction in the expression of the MEX3B gene. The Cas nuclease or the nucleic acid encoding the Cas nuclease and the guide RNA or the DNA encoding the guide RNA may be transferred into cells by various methods known in the art, examples of which include, but are not limited to, microinjection, electroporation, DEAE-dextran treatment, lipofection, nanoparticles-mediated transfection, protein transduction domain-mediated transduction, virus-mediated gene delivery, and PEG-mediated transfection into protoplasts. The Cas nuclease or the nucleic acid encoding the Cas nuclease and the guide RNA may be transferred into an organism by various methods known in the art, such as injection or other methods for administration of genes or proteins. The nucleic acid encoding the Cas nuclease or the Cas protein and the guide RNA may be transferred separately into cells, or a conjugate of the nucleic acid encoding the Cas nuclease or the Cas protein and the guide RNA may be transferred into cells. The Cas nuclease may also be fused with a protein transduction domain, such as Tat, for its efficient delivery into cells. Preferably, eukaryotic cells or organisms are co-transfected or sequentially transfected with Cas9 nuclease and the guide RNA. The sequential transfection may comprise performing first transfection with the nucleic acid encoding the Cas nuclease and then performing second transfection with the naked guide RNA. As a non-limiting example, the second transfection is preferably performed 3, 6, 12, 18, or 24 hours after the first transfection. A guide RNA expression unit may also be used to express the guide RNA. The guide RNA expression unit is preferably a CRISPR-Cas9 transcription unit comprising the target sequence (a partial sequence of the MEX3B gene) and the guide RNA. Such a transcription unit preferably comprises a promotor region (an RNA polymerase III promoter, such as a promoter selected from U6 promoter and H1 promoter) for the expression of the guide RNA; the target gene (MEX3B gene); and the guide RNA, and more preferably comprises the promoter; a sequence complementary to the target sequence (at least a partial sequence of the MEX3B gene); and the guide RNA, in which the promoter, the sequence, and the guide RNA are seamlessly liked together. To prevent CRISPR-Cas nuclease offtargeting, a Cas9 mutant (nickase) may also be used that cleaves only one of the two DNA strands. The single strand-cleaving Cas9 mutant may be, for example, Cas9(D10A). For example, the single strand-cleaving Cas9 mutant may be used in combination with a guide RNA having a target sequence complementary to one of target DNA strands and another guide RNA having a target sequence complementary to the other strand very close to the one strand. In this case, one strand can be cleaved in a way specific for 20 nucleotides, while the other can be cleaved in a way specific for 20 nucleotides, so that the DNA can be cleaved in a way specific for 40 nucleotides, which will significantly increase the target specificity.

The dosage of the artificial nuclease or the nucleic acid encoding the artificial nuclease, used as an active component, may be usually in the range of about 0.1 μg to about 10 mg per kg body weight per dose.

Aptamer or Antibody That Selectively Binds to MEX3B Protein

The substance inhibiting an MEX3B protein may be any substance that inhibits the function of the MEX3B protein, such as a polymer compound (e.g., a nucleic acid, such as an aptamer), an antibody, or a low-molecular-weight compound. A preferred mode of the substance inhibiting an MEX3B protein is an aptamer that selectively binds to the MEX3B protein. The prophylactic or therapeutic agent for at least one cancer selected from the group consisting of pancreatic cancer, lung cancer, colorectal cancer, cholangiocarcinoma, and liver cancer may comprise such an aptamer. The term "aptamer" refers to a nucleic acid drug that comprises a single-stranded RNA or DNA and has a three-dimensional structure capable of binding to a target protein to inhibit the function of the target protein. The aptamer has a high ability to bind to the target protein, has a high specificity for the target protein, has low immunogenicity, and has high storage stability. The aptamer can be produced by chemical synthesis. The aptamer may have any length that allows it to selectively bind to the MEX3B protein. The aptamer that selectively binds to the MEX3B protein is preferably 15 to 60 nucleotides in length, more preferably 20 to 50 nucleotides in length, even more preferably 25 to 47 nucleotides in length, most preferably 26 to 45 nucleotides in length. The aptamer that selectively binds to the MEX3B protein may be obtained by SELEX (systematic evolution of ligands by exponential enrichment) technique.

Another preferred mode of the substance inhibiting an MEX3B protein is an antibody that selectively binds to the MEX3B protein. The prophylactic or therapeutic agent for at least one cancer selected from the group consisting of pancreatic cancer, lung cancer, colorectal cancer, cholangiocarcinoma, and liver cancer may comprise such an antibody. The antibody that selectively binds to the MEX3B protein may be a polyclonal or monoclonal antibody. The polyclonal antibody may be prepared by a process that includes immunizing animals with an antigen; obtaining the serum from the immunized animals; and subjecting the serum to separation and purification. The monoclonal antibody may be prepared by a process that includes immunizing animals with an antigen; obtaining antibody-producing cells from the immunized animals; fusing the antibody-producing cells with myeloma cells to form hybridoma cells; culturing the hybridoma cells or administering the hybridoma cells to animals to allow them to develop ascites cancer; and subjecting the culture or the ascitic fluid to separation and purification. The monoclonal antibody may also be prepared by a process that includes fusing the antibody-producing cells with myeloma cells derived from non-human mammals to form hybridoma cells; culturing the hybridoma cells or administering the hybridoma cells to animals to allow them to develop ascites cancer; and subjecting the culture or the ascitic fluid to separation and purification. The antibody-producing cells may be spleen cells, lymph node cells, or antibody-producing cells in peripheral blood. In particular, the antibody-producing cells are preferably spleen cells.

The antibody to be administered to a human is preferably a human reshaped antibody or a humanized antibody. The human reshaped antibody or humanized antibody may be prepared using mammals, such as transgenic mice. The human reshaped antibody is described in, for example, Morrison, S. L. et al., Proc. Natl. Acad. Sci. USA, 81:6851-6855 (1984) and Hiroshi Noguchi, Igaku no ayumi 167:457-462 (1993) (in Japanese). The humanized chimeric antibody may be prepared using genetic recombination to combine the V region of a mouse antibody with the C region of a human antibody. The humanized antibody may be prepared from a mouse monoclonal antibody by substituting a human antibody-derived sequence for a non-complementaritydetermining region (non-CDR) of the mouse monoclonal antibody. The antibody may be immobilized on an insoluble carrier, such as a solid-phase carrier, to form an immobilized antibody before use, or may be labeled with a labeling substance to form a labeled antibody before use. Such immobilized and labeled antibodies all fall within the scope of the present invention.

The antibody described above may be an antibody that selectively (preferably specifically) binds to the MEX3B protein to inhibit the function of the MEX3B protein. The prophylactic or therapeutic agent for at least cancer selected from the group consisting of pancreatic cancer, lung cancer, colorectal cancer, cholangiocarcinoma, and liver cancer may comprise the antibody that selectively (or specifically) binds to the MEX3B protein to inhibit the function of the MEX3B protein.

The prophylactic or therapeutic agent according to the first aspect may be prepared in the form of a pharmaceutical composition comprising: the antibody described above as an active component; and a pharmaceutically acceptable carrier, diluent (e.g., an immunogenic adjuvant), stabilizer, or excipient. The prophylactic or therapeutic agent comprising the antibody may be sterilized by filtration and lyophilized. The lyophilized product may be formulated into a dosage form in a vial for administration or into a stabilized aqueous preparation.

Regarding a specific mode of the prophylactic or therapeutic agent according to the first aspect, the pancreatic cancer may be pancreatic adenocarcinoma, the lung cancer may be non-small cell lung cancer, the colorectal cancer may be colon cancer, and the liver cancer may be hepatocellular cancer. The prophylactic or therapeutic agent may be administered to a subject (e.g., a patient, an asymptomatic patient) by a method known to those skilled in the art, such as intra-arterial injection, intravenous injection, or subcutaneous injection. The dosage may vary depending on the body weight and age of the subject (e.g., patient, asymptomatic patient), the administration route, and other factors. Those skilled in the art can select an appropriate dosage depending on them. The dosage of the antibody used as an active component may be usually in the range of about 0.1 μg to about 100 mg per kg body weight per dose.

Regarding the prophylactic or therapeutic agent according to the first aspect, the at least one cancer may or may not be refractory to other anticancer agents. The prophylactic or therapeutic agent according to the first aspect can act through a different active site or a different action mechanism than such other anticancer agents, and thus can be effective against such refractory cancer. Otherwise, the prophylactic or therapeutic agent according to the first aspect can be effective in reducing the dosage of other conventional anticancer agents (specifically, effective in reducing the side effects of other conventional anticancer agents or improving medication compliance). In other words, the prophylactic or therapeutic agent according to the first aspect may or may not be an agent to be administered to a patient suffering from the refractory cancer mentioned above. The anticancer agent other than the prophylactic or therapeutic agent according to the first aspect may be any type. Preferably, the anticancer agent other than the prophylactic or therapeutic agent according to the first aspect is at least one selected from the group consisting of an immune checkpoint inhibitor and a pyrimidine antimetabolite. The immune checkpoint inhibitor may be an agent that inhibits the function of an immune checkpoint molecule (e.g., binding between immune checkpoint molecules, such as binding between a receptor and its ligand). Examples of the immune checkpoint molecule include receptors, such as PD-1 and CTLA4, and ligands, such as PD-L1, PD-L2, and CD80/86. The immune checkpoint inhibitor may be a substance (e.g., an antibody, an aptamer) that selectively (preferably specifically) binds to such an immune checkpoint molecule. Specific examples of the immune checkpoint inhibitor include anti-PD-1 antibodies, anti-CTLA4 antibodies, anti-PD-L1 antibodies, anti-PD-L2 antibodies, and anti-CD80/86 antibodies.

The pyrimidine antimetabolite may be an agent that inhibits in vivo synthesis of nucleic acid or an agent that can be converted into an agent that inhibits in vivo synthesis of nucleic acid. Specific examples of the pyrimidine antimetabolite include gemcitabine (abbreviated as Gem), cytarabine, capecitabine, TS-1 (registered trademark), tegafur-gimeracil-oteracil potassium (S-1), tegafur-uracil, and fluorouracil.

The prophylactic or therapeutic agent according to the first aspect may be for use in combination with the other anticancer agent described above to form a combination drug (pharmaceutical combination). In this case, the prophylactic or therapeutic agent according to the first aspect is effective in reducing the dosage of the other anticancer agent (specifically, effective in reducing the side effects of the other conventional anticancer agent or improving medication compliance). In a case where the at least one cancer is refractory to the other anticancer agent described above, the prophylactic or therapeutic agent according to the first aspect may also be for use in combination with the other anticancer agent to form a combination drug to be administered to a patient suffering from such refractory cancer.

The prophylactic or therapeutic agent according to the first aspect may be administered orally or parenterally and administered systemically or locally. Parenteral administration methods include intratumoral injection, intravenous injection, such as drip infusion, intraperitoneal injection, subcutaneous injection, and intramuscular injection. A suitable administration method may be selected depending on the age and condition of the subject (e.g., patient, asymptomatic patient). The dosage varies depending on the age, the route of administration, and the frequency of administration. Those skilled in the art can select an appropriate dosage depending on them. The preparation in a form suitable for parenteral administration may further comprise an additive, such as a stabilizer, a buffer, a preservative, or an isotonic agent, and a pharmaceutically acceptable carrier or additive. Examples of such a carrier and additive include, but are not limited to, water, organic solvents, polymer compounds (e.g., collagen, polyvinyl alcohol), stearic acid, human serum albumin (HSA), mannitol, sorbitol, lactose, and surfactants.

Prophylactic or Therapeutic Agent for at Least One Cancer Selected from the Group Consisting of Pancreatic Cancer, Lung Cancer, Colorectal Cancer, Cholangiocarcinoma, and Liver Cancer and for Use in Combination with the Prophylactic or Therapeutic Agent according to the First Aspect to Form Combination Drug

The prophylactic or therapeutic agent according to the second aspect is for at least one cancer selected from the group consisting of pancreatic cancer, lung cancer, colorectal cancer, cholangiocarcinoma, and liver cancer and for use in combination with the prophylactic or therapeutic agent according to the first aspect to form a combination drug. The prophylactic or therapeutic agent according to the second aspect may comprise any anticancer agent as an active component. The prophylactic or therapeutic agent according to the second aspect preferably comprises an anticancer agent that acts through a different active site or a different action mechanism than the prophylactic or therapeutic agent according to the first aspect, and more preferably comprises at least one anticancer agent selected from the group consisting of an immune checkpoint inhibitor and a pyrimidine antimetabolite. Specific and preferred examples of the immune checkpoint inhibitor and the pyrimidine antimetabolite are as shown above. The prophylactic or therapeutic agent according to the second aspect may be administered orally or parenterally and administered systemically or locally. Parenteral administration methods include intratumoral injection, intravenous injection, such as drip infusion, intraperitoneal injection, subcutaneous injection, and intramuscular injection. A suitable administration method may be selected depending on the age and condition of the subject (e.g., patient, asymptomatic patient). The dosage varies depending on the age, the route of administration, and the frequency of administration. Those skilled in the art can select an appropriate dosage depending on them. The preparation in a form suitable for parenteral administration may further comprise an additive, such as a stabilizer, a buffer, a preservative, or an isotonic agent, and a pharmaceutically acceptable carrier or additive. Examples of such a carrier and additive include, but are not limited to, water, organic solvents, polymer compounds (e.g., collagen, polyvinyl alcohol), stearic acid, human serum albumin (HSA), mannitol, sorbitol, lactose, and surfactants.

Combination Drug

The combination drug according to the third aspect comprises the prophylactic or therapeutic agent according to the first aspect and an additional anticancer agent other than the prophylactic or therapeutic agent. The combination drug according to the third aspect is effective in reducing the dosage of the additional anticancer agent (specifically, effective in reducing the side effects of the additional anticancer agent or improving medication compliance). The combination drug according to the third aspect may be such that the at least one cancer is refractory to the additional anticancer agent and that it is to be administered to a patient suffering from such refractory cancer. The additional anticancer agent may be any type. The additional anticancer agent is preferably an anticancer agent that acts through a different active site or a different action mechanism than the prophylactic or therapeutic agent according to the first aspect and is more preferably at least one anticancer agent selected from the group consisting of an immune checkpoint inhibitor and a pyrimidine antimetabolite. Specific and preferred examples of the immune checkpoint inhibitor and the pyrimidine antimetabolite are as shown above.

The combination drug according to the third aspect may comprise a mixture of the prophylactic or therapeutic agent according to the first aspect and the additional anticancer agent for administration or may comprise the prophylactic or therapeutic agent according to the first aspect and the additional anticancer agent separately for separate and simultaneous administration. Regarding the combination drug according to the third aspect, the route of administration of the prophylactic or therapeutic agent according to the first aspect and the route of administration of the additional anticancer agent may be the same or different, and the dosages of them may also be the same or different. The combination drug according to the third aspect may be administered orally or parenterally and administered systemically or locally. Parenteral administration methods include intratumoral injection, intravenous injection, such as drip infusion, intraperitoneal injection, subcutaneous injection, and intramuscular injection. A suitable administration method may be selected depending on the age and condition of the subject (e.g., patient, asymptomatic patient). The dosage varies depending on the age, the route of administration, and the frequency of administration. Those skilled in the art can select an appropriate dosage depending on them. The preparation in a form suitable for parenteral administration may further comprise an additive, such as a stabilizer, a buffer, a preservative, or an isotonic agent, and a pharmaceutically acceptable carrier or additive. Examples of such a carrier and additive include, but are not limited to, water, organic solvents, polymer compounds (e.g., collagen, polyvinyl alcohol), stearic acid, human serum albumin (HSA), mannitol, sorbitol, lactose, and surfactants.

Method for Screening for Prophylactic or Therapeutic Agent for at Least One Cancer Selected from the Group Consisting of Pancreatic Cancer, Lung Cancer, Colorectal Cancer, Cholangiocarcinoma, and Liver Cancer

The screening method according to the fourth aspect comprises screening test substances for a prophylactic or therapeutic agent for at least one cancer selected from the group consisting of pancreatic cancer, lung cancer, colorectal cancer, cholangiocarcinoma, and liver cancer using at least one indicator selected from the group consisting of a reduction in MEX3B gene expression or MEX3B protein expression and a reduction in MEX3B protein function.

In the screening method according to the fourth aspect, the at least one cancer may or may not be refractory to other anticancer agents. The prophylactic or therapeutic agent, which is to be found by the screening, may or may not be the agent for use in combination with the other anticancer agent described above to form a combination drug. The other anticancer agent may be any type. The other anticancer agent is preferably an anticancer agent that acts through a different active site or a different action mechanism than the prophylactic or therapeutic agent and is more preferably at least one anticancer agent selected from the group consisting of an immune checkpoint inhibitor and a pyrimidine antimetabolite.

The screening method according to the fourth aspect preferably comprises screening test substances using, as an indicator, a reduction in MEX3B gene expression. The MEX3B protein function may be the function of promoting the development of at least one cancer selected from the group consisting of colorectal cancer, cholangiocarcinoma, and liver cancer; the function of inducing the expression by p53; the function of inducing cellular senescence; the function of binding to various mRNAs of the inflammatory cytokine or chemokine genes to control the function of the mRNAs (namely the translation to proteins) or the stability of the mRNAs; or the function of inducing the expression of the inflammatory cytokines or chemokines. The reduction may be at any statistically significant level. The reduction in the expression or the function of the MEX3B gene or the MEX3B protein in the presence of a test substance is preferably to 1/2 or less, more preferably to 1/4 or less, even more preferably to 1/10 or less of that in the absence of the test substance (e.g., in a system before the administration of the test substance, such as a wild type system, or in a negative control system, such as a control system in which a substance having no effect on the expression or the function of the MEX3B gene or the MEX3B protein is administered). Most preferably, no expression or function of the MEX3B gene or the MEX3B protein is observed in the presence of the test substance.

The screening method may be any type, such as an in vivo, in vitro, or in silico screening method, as long as it is performed using the specified indicator. A preferred example of the screening method comprises culturing MEX3B gene-expressing cells in the presence of a test substance and culturing MEX3B gene-expressing cells in the absence of the test substance; and determining whether a reduction in MEX3B gene expression or MEX3B protein expression or a reduction in MEX3B protein function is observed as an indicator depending on the presence or absence of the test substance for screening for a prophylactic or therapeutic agent for at least one cancer selected from the group consisting of pancreatic cancer, lung cancer, colorectal cancer, cholangiocarcinoma, and liver cancer. The screening may be performed using the full-length MEX3B protein or using part of the MEX3B protein (e.g., at least any one domain characteristic of the MEX3B protein). Such a domain may be any RNA-binding or proteinbinding domain of the MEX3B protein. More specifically, such a domain may be KH domain or RING finger domain.

The expression of the MEX3B gene in various human tissues can be detected even by in silico investigation based on the MEX3B gene sequence information. Probes or primers having part or the whole of the gene sequence may also be used to detect in vivo or in vitro expression of the MEX3B gene in various human tissues. Conventional methods, such as RT-PCR, Northern blotting, and Southern blotting, may be used to detect the MEX3B gene expression. Conventional methods, such as RT-PCR, Northern blotting, and Southern blotting, may also be used to determine the level of expression of mRNA of the MEX3B gene.

PCR may be performed using any primers capable of specifically amplifying only the MEX3B gene, which may be selected as appropriate based on the MEX3B gene sequence information. For example, an oligonucleotide comprising at least 10 contiguous nucleotides of the sequence of the MEX3B gene or the expression control region of the MEX3B gene and an antisense oligonucleotide having a sequence complementary to the oligonucleotide may be used as the probes or primers. More specifically, an oligonucleotide comprising 10 to 60, preferably 10 to 40, contiguous nucleotides of the sequence of the MEX3B gene or the expression control region of the MEX3B gene and an antisense oligonucleotide having a sequence complementary to the oligonucleotide may be used as the probes or primers.

The oligonucleotide and the antisense oligonucleotide may be produced by a conventional method using a DNA synthesizer. The oligonucleotide or the antisense oligonucleotide may be, for example, a sense primer corresponding to a 5' end part of the mRNA sequence to be detected or an antisense primer corresponding to a 3' end part of the mRNA sequence to be detected. Examples of each of the sense primer and the antisense primer include oligonucleotides that are not significantly variable in melting temperature (Tm) or length and are about 10 to about 60 nucleotides in length, preferably about 10 to about 40 nucleotides in length. In the present invention, derivatives of the oligonucleotides may also be used, such as methyl or phosphorothioate derivatives of the oligonucleotides.

The level of expression of the MEX3B protein may be measured by conventional immunoassay, such as Western blotting or ELISA, using the antibody shown later. Specifically, the expression level may be measured by conventional methods known to those skilled in the art, such as those described in Molecular Cloning 2nd Edition or Current Protocols in Molecular Biology. Whether there is a reduction in MEX3B protein function may be analyzed by determining whether or how much the MEX3B protein has the ability to bind to mRNA or by determining whether or how much the mRNA, to which the MEX3B protein binds, performs its function. Any suitable analysis, such as a competitive inhibition test, may be used to determine whether or how much the MEX3B protein has the ability to bind to mRNA. A conventional immunoassay, such as Western blotting or ELISA, may be used to measure the protein expression level for use to determine whether or how much the mRNA, to which the MEX3B protein binds, performs its function. For example, the expression level may be measured by conventional methods known to those skilled in the art, such as those described in Molecular Cloning 2nd Edition or Current Protocols in Molecular Biology.

Any test substances may be subjected to the screening method according to the fourth aspect. The test substances may be any type, examples of which include nucleic acid molecules, antibodies, low-molecular-weight synthetic compounds, compounds found in natural product extracts, and synthetic peptides. The test substances may also be artificial nucleases for genome editing as shown below. The test substances may also be derived from a compound library, a phage display library, or a combinatorial library. The construction of the compound library is known to those skilled in the art, and a commercially available compound library may also be used. The test substances are preferably low-molecular-weight compounds (e.g., derived from a compound library), nucleic acid molecules, artificial nucleases for genome editing, or antibodies, among which nucleic acid molecules or antibodies are more preferred, since they have high specificity for the MEX3B gene or protein, and nucleic acid molecules having a sequence complementary to an oligonucleotide sequence in the MEX3B gene (in the CDS or UTR of the exon or in the intron) or in the expression control region of the MEX3B gene, or aptamers or antibodies that selectively bind to the MEX3B protein are most preferred.

Method for Preventing or Treating at Least One Cancer Selected from the Group Consisting of Pancreatic Cancer, Lung Cancer, Colorectal Cancer, Cholangiocarcinoma, and Liver Cancer

The present invention may or may not be directed to a method for preventing or treating at least one cancer selected from the group consisting of pancreatic cancer, lung cancer, colorectal cancer, cholangiocarcinoma, and liver cancer. The method for preventing or treating at least one cancer selected from the group consisting of pancreatic cancer, lung cancer, colorectal cancer, cholangiocarcinoma, and liver cancer according to the fifth aspect comprises administering the prophylactic or therapeutic agent according to the first aspect to a subject. The method for preventing or treating at least one cancer selected from the group consisting of pancreatic cancer, lung cancer, colorectal cancer, cholangiocarcinoma, and liver cancer according to the sixth aspect comprises administering the prophylactic or therapeutic agent according to the first aspect in combination with the additional anticancer agent described above. The subject may be an animal. The subject is preferably a vertebrate, more preferably a mammal, such as human, pig, cow, mouse, or rat, even more preferably a human, most preferably a symptomatic human patient. Specific and preferred examples of the administration method, the dosage, and other conditions are as shown above.

EXAMPLES

Hereinafter, the present invention will be more specifically described with reference to examples, which are not intended to limit the scope of the present invention.

Antisense Oligonucleotides

The antisense oligonucleotides shown below were used in the examples shown below. Table 1 is a list of gapmer 89 (SEQ ID NO: 8), gapmer 89-2 (SEQ ID NO: 9), and gapmer NC.

[Table 1]

| Antisense oligonucleotide | SEQ ID NO: | Gapmer sequence | Oligonucleotide length | Target sequence | Target region (Human pre-mRNA) | Target region (Mouse gene) | Target region |
|---|---|---|---|---|---|---|---|
| Gapmer 89 | 8 | GAGCGGATACTACAG#C | 16 | GCTGTAGTATCCGCTC | 3139-3154 | 2195-2210 | 3' UTR |
| Gapmer 89-2 | 9 | G#CGGATACTACAGCTT | 16 | AAGCTGTAGTATCCGC | 3137-3152 | 2193-2208 | 3' UTR |
| Gapmer NC | 10 | AACACGTCTATACG#C | 15 | Absent | Absent | Absent | Absent |
| #:Cytidine methylated at position 5 to form LNA | | | | | | | |

As shown in the table, gapmer 89-2 is an antisense oligonucleotide having a target sequence shifted two residues toward the 5' terminus from the target sequence of gapmer 89. Gapmer NC is a negative control (NC) gapmer that targets a sequence not found in any genome.

Each of the gapmer-type antisense oligonucleotides had two LNA (2',4'-BNA) nucleotides at each end and normal DNA nucleotides were employed as the nucleotides between the both ends, while each phosphodiester bond between nucleotides was replaced by a phosphorothioate bond.

Example 1

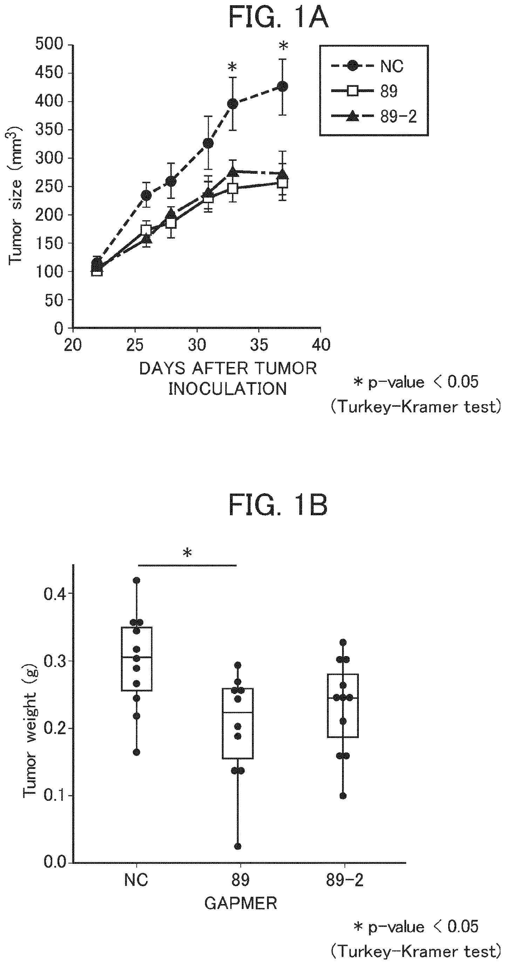

Nude mice (Balb/c-nu/nu, female, 7 weeks old) were inoculated with human pancreatic cancer cells PK1 ($3 \times 10^6$ cells/mouse), and 23, 26, 28, 31, 33, and 35 days after the inoculation, gapmer 89 (20 μg/200 μL micelles (50% cRGD)/mouse) or gapmer 89-2 (20 μg/200 μL micelles (50% cRGD)/mouse) was administered to the mice via tail vein injection, while 22, 26, 28, 31, 33, 35, and 37 days after the inoculation, the long and short diameters of the tumors were measured with a caliper and used to calculate the tumor volume (tumor size) from the formula: (long diameter) $\times$ (short diameter)$^2$/2 (mm$^3$) (hereinafter the same shall apply). On day 37, the tumor was removed and weighed (tumor weight (g)). The micelles were polymer micelles at most 100 nm in particle size having a cRGD ligand molecule and having a core-shell structure formed by the antisense oligonucleotide and a block copolymer comprising PEG and poly(amino acid). The same test procedure was performed using gapmer NC (negative control (NC)), which targeted a sequence not found in any genome. The results are shown in FIGS. 1A and 1B.

FIGS. 1A and 1B are graphs showing the results of the pancreatic cancer cell (PK1) growth inhibition test of the antisense oligonucleotides against MEX3B. In the graphs, the error bars indicate the standard errors. The symbol * indicates p-value < 0.05 by Tukey-Kramer test. The results shown in FIGS. 1A and 1B indicate that the administrations of gapmer 89 and gapmer 89-2 reduced (especially reduced significantly on day 33 and later) the volume and weight of the tumor as compared to the administration of gapmer NC. This means that gapmer 89 and gapmer 89-2 are substances that reduce the expression of the MEX3B gene or the MEX3B protein and are effective in inhibiting pancreatic cancer cell growth. The results suggest that pancreatic cancer cell growth can be inhibited by inhibiting the expression of the MEX3B gene, not depending on the target sequence to be knocked down in the MEX3B gene.

Moreover, instead of the micelles administered via tail vein, a mixture of gapmer 89 and in vivo-jetPEI (registered trademark) was intraperitoneally administered in the dose:
gapmer 8 μg/in vivo-jetPEI (registered trademark) 80 μL/mouse. As a result, similar to that shown in FIGS. 1A and 1B, the administration of gapmer 89 significantly reduced the volume and weight of the tumor as compared to the administration of gapmer NC. The results suggest that pancreatic cancer cell growth can be inhibited by inhibiting the expression of the MEX3B gene, not depending on how to administer the substance that reduces expression of the MEX3B gene or the MEX3B protein or not depending on what route is used to administer the substance.

Example 2

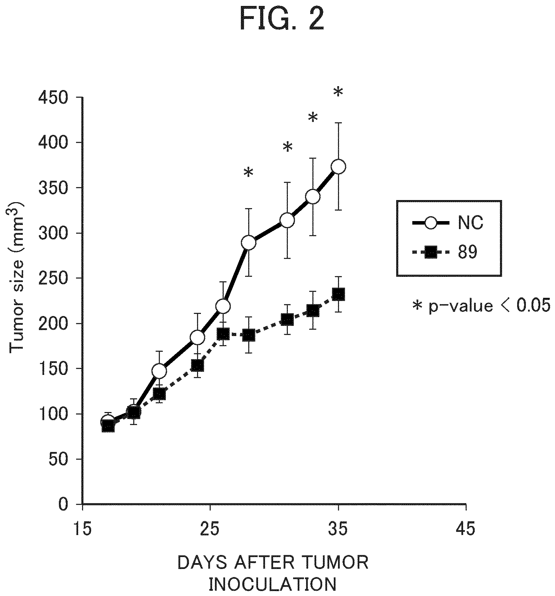

Nude mice (Balb/c-nu/nu, female, 7 weeks old) were inoculated with human pancreatic cancer cells AsPC1 ($3 \times 10^6$ cells/mouse), and 17, 19, 21, 24, 26, 28, 31, 33, and 35 days after the average tumor volume reached approximately 80 mm$^3$, gapmer 89 (20 μg/200 μL micelles (50% cRGD)/mouse) was administered to the mice via tail vein injection, while 17, 19, 21, 24, 26, 28, 31, 33, and 35 days after the inoculation, the long and short diameters of the tumors were measured with a caliper and used to calculate the tumor volume (tumor size) (mm$^3$). The micelles were polymer micelles at most 100 nm in particle size having a cRGD ligand molecule and having a core-shell structure formed by the antisense oligonucleotide and a block copolymer comprising PEG and poly(amino acid). The same test procedure was performed using gapmer NC (negative control (NC)), which targeted a sequence not found in any genome. The results are shown in FIG. 2.

FIG. 2 is a graph showing the results of the pancreatic cancer cell (AsPC1) growth inhibition test of the antisense oligonucleotide against MEX3B. In the graph, the error bars indicate the standard errors. The symbol * indicates p-value < 0.05 by t-test. The results shown in FIG. 2 indicate that the administration of gapmer 89 reduced (especially reduced significantly on day 28 and later) the tumor volume as compared to the administration of gapmer NC. This means that gapmer 89 is a substance that reduces expression of the MEX3B gene or the MEX3B protein and is effective in inhibiting pancreatic cancer cell growth.

Example 3

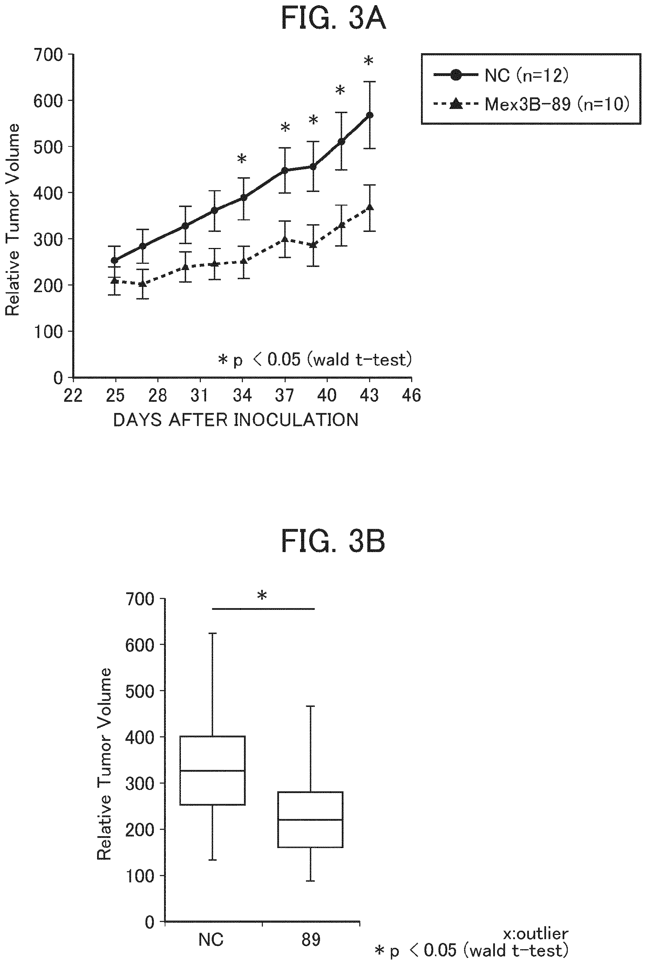

Nude mice (Balb/c-nu/nu, female, 7 weeks old) were inoculated with human non-small cell lung cancer cells A549 ($3.0 \times 10^6$ cells/mouse), and 25, 27, 32, 34, 37, 39, 41, and 43 days after the inoculation, gapmer 89 (20 μg/200 μL micelles (50% cRGD)/mouse) was administered to the mice via tail vein injection, while 25, 27, 32, 34, 37, 39, 41, and 43 days after the inoculation, the long and short diameters of the tumors were measured with a caliper and used to calculate the tumor volume (tumor size). On day 43, the tumor was removed and weighed (tumor weight (g)). The micelles were polymer micelles at most 100 nm in particle size having a cRGD ligand molecule and having a core-shell structure formed by the antisense oligonucleotide and a block copolymer comprising PEG and poly(amino acid). The same test procedure was performed using gapmer NC (negative control (NC)), which targeted a sequence not found in any genome. The results are shown in FIGS. 3A and 3B.

FIGS. 3A and 3B are graphs showing the results of the non-small cell lung cancer cell growth inhibition test of the antisense oligonucleotide against MEX3B. In the graphs, the error bars indicate the standard errors. The symbol * indicates p-value < 0.05 by Wald t-test. The results shown in FIGS. 3A and 3B indicate that the administration of gapmer 89 reduced (especially reduced significantly on day 34 and later) the volume and weight of the tumor as compared to the administration of gapmer NC. This means that gapmer 89 is a substance that reduces expression of the MEX3B gene or the MEX3B protein and is effective in inhibiting non-small cell lung cancer cell growth.

Example 4

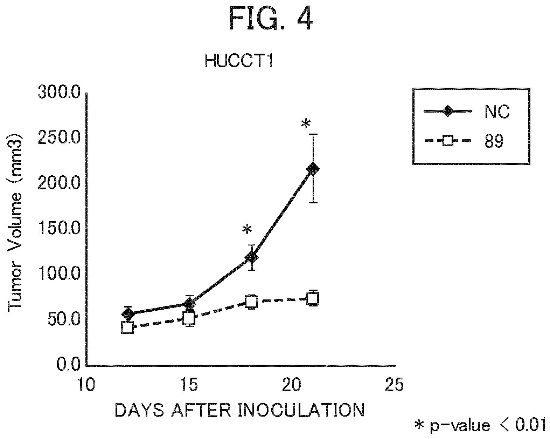

Nude mice (Balb/c-nu/nu, female, 7 weeks old) were inoculated with human cholangiocarcinoma cells HUCCT1 ($3.0 \times 10^6$ cells/mouse), and from 12 days after the inoculation, gapmer 89 together with in vivo-jetPEI (registered trademark) was intratumorally administered in the dose: gapmer 5 μg/in vivo-jetPEI (registered trademark) 80 μL/mouse at a frequency of once per 3 days, while the long and short diameters of the tumors were measured with a caliper and used to calculate the tumor volume (tumor size) from the formula: (long diameter) $\times$ (short diameter)$^2$/2 (mm$^3$). The same test procedure was performed using gapmer NC (negative control (NC)), which targeted a sequence not found in any genome. The results are shown in FIG. 4.

FIG. 4 is a graph showing the results of the cholangiocarcinoma cell growth inhibition test of the antisense oligonucleotide against MEX3B. In the graph, the error bars indicate the standard errors. The symbol * indicates p-value < 0.01. The results shown in FIG. 4 indicate that the administration of gapmer 89 reduced (especially reduced significantly on day 18 and later) the volume and weight of the tumor as compared to the administration of gapmer NC. This means that gapmer 89 is a substance that reduces expression of the MEX3B gene or the MEX3B protein and is effective in inhibiting cholangiocarcinoma cell growth.

Example 5

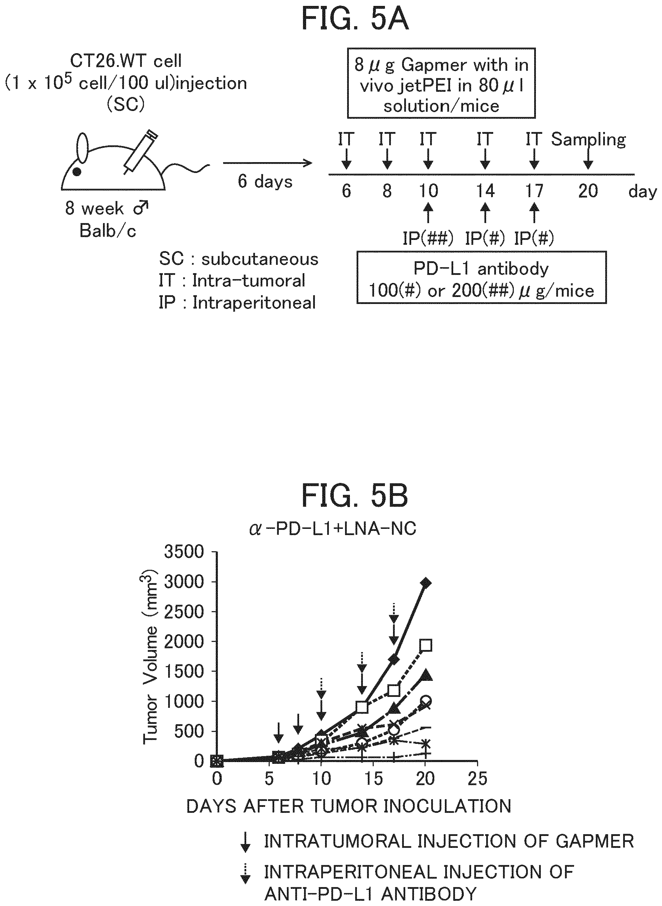

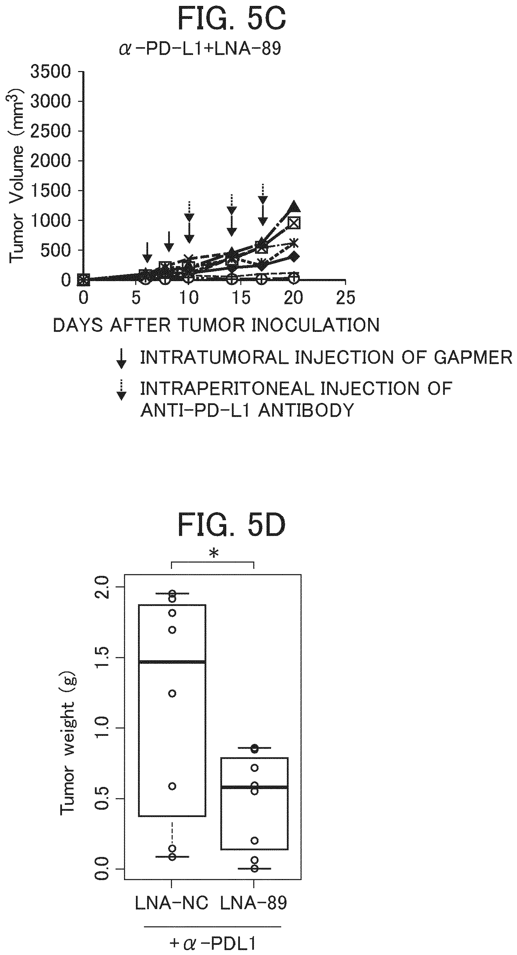

Wild-type mice (Balb/c, male, 8 weeks old) were inoculated with mouse colorectal cancer cell line CT26 cells ($1.0 \times 10^5$ cells/mouse), and 6, 8, 10, 14, and 17 days after the inoculation, gapmer 89 (gapmer 8 μg/80 μL in vivo-jetPEI (registered trademark)/mouse) was intratumorally administered, while an anti-PD-L1 antibody was intraperitoneally administered 10 days (200 μg/mouse), 14 days (100 μg/mouse), and 17 days (100 μg/mouse) after the inoculation, and 6, 8, 10, 14, 17, and 20 days after the inoculation, the longitudinal and transverse diameters of the tumors were measured with a caliper and used to calculate the tumor volume (mm$^3$). On day 20, the tumor was removed and weighed (tumor weight (g)). The same test procedure was performed using gapmer NC (negative control (NC)), which targeted a sequence not found in any genome. The results are shown in FIGS. 5B, 5C, and 5D.

FIG. 5A is a diagram showing the outline of the procedure of the colorectal cancer cell growth inhibition test in which the antisense oligonucleotide against MEX3B was administered in combination with the immune checkpoint inhibitor. FIG. 5B is a graph showing the results (tumor volumes) of the growth inhibition test in which gapmer NC was administered in combination with the immune checkpoint inhibitor. FIG. 5C is a graph showing the results (tumor volumes) of the growth inhibition test in which the antisense oligonucleotide against MEX3B was administered in combination with the immune checkpoint inhibitor. FIG. 5D is a graph showing the results (tumor weights) of the growth inhibition test in which the antisense oligonucleotide against MEX3B was administered in combination with the immune checkpoint inhibitor. In the graphs, the error bars indicate the standard errors. The symbol * indicates p-value < 0.05 by Wald t-test.

A comparison between the results shown in FIGS. 5B and 5C and the results shown in FIG. 5D indicate that the administration of gapmer 89 in combination with the anti-PD-L1 antibody significantly reduced the volume and weight of the tumor as compared to the administration of gapmer NC in combination with the anti-PD-L1 antibody. This suggests that the administration of gapmer 89, which is a substance that reduces expression of the MEX3B gene or the MEX3B protein, in combination with an immune checkpoint inhibitor is effective in further inhibiting colorectal cancer cell growth no matter whether the immune checkpoint inhibitor is effective in inhibiting cancer cell growth.

Example 6

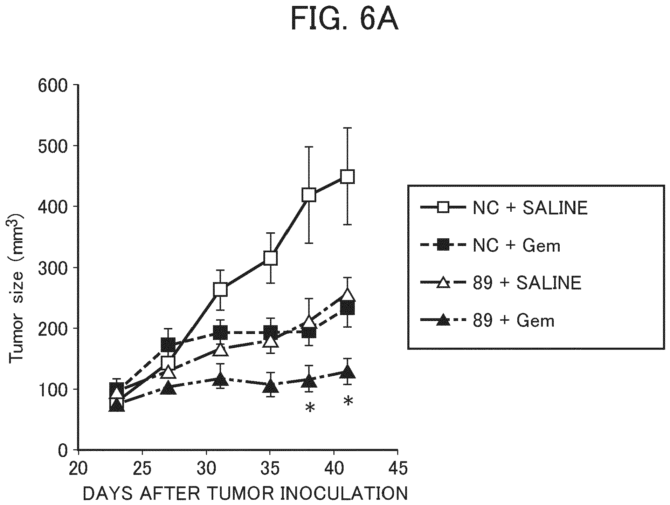

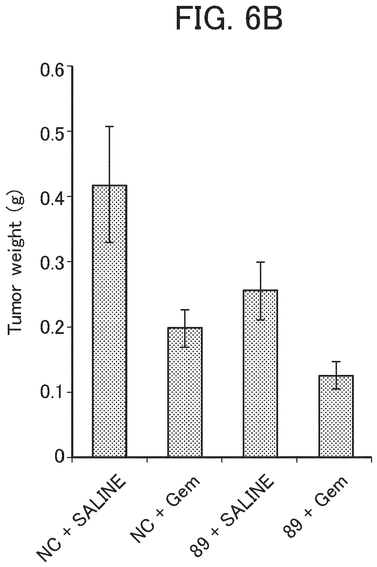

Nude mice (Balb/c-nu/nu, female, 7 weeks old) were inoculated with human pancreatic cancer cells PK1 ($3 \times 10^6$ cells/mouse). From 23 to 39 days after the inoculation, gapmer 89 (20 μg/200 μL micelles (50% cRGD)/mouse) was administered via tail vein injection every other day, while gemcitabine (50 mg/kg) was intraperitoneally administered 23, 27, 31, and 35 days after the inoculation, and 23, 27, 31, 35, 38, and 41 days after the inoculation, the longitudinal and transverse diameters of the tumors were measured with a caliper and used to calculate the tumor volume (tumor size) ($mm^3$). On day 41, the tumor was removed and weighed (tumor weight (g)). The same test procedure was performed using gapmer NC (negative control (NC)), which targeted a sequence not found in any genome. The results are shown in FIGS. 6A and 6B.

FIGS. 6A and 6B are graphs showing the results of the pancreatic cancer cell growth inhibition test in which the antisense oligonucleotide against MEX3B was administered in combination with the pyrimidine antimetabolite. In the graphs, the error bars indicate the standard errors. The results shown in FIG. 6A indicate that the administration of gapmer 89 in combination with gemcitabine (Gem) reduced the tumor volume more than the administration of gapmer NC in combination with Gem or the administration of gapmer 89 alone. Moreover, at least 38 days after the inoculation, the reduction in the tumor volume by the administration of gapmer 89 in combination with Gem was significantly greater than that by the administration of gapmer NC in combination with Gem or the administration of gapmer 89 alone with p-value < 0.05 in t-test.

The results shown in FIG. 6B indicate that the administration of gapmer 89 in combination with Gem reduced the tumor weight more than the administration of gapmer NC in combination with Gem or the administration of gapmer 89 alone. Moreover, the reduction in the tumor weight by the administration of gapmer 89 in combination with Gem was significantly greater than that by the administration of gapmer NC in combination with Gem or the administration of gapmer 89 alone with p value < 0.05 in t-test. This suggests that the administration of gapmer 89, which is a substance that reduces expression of the MEX3B gene or the MEX3B protein, in combination with a pyrimidine antimetabolite is effective in further inhibiting pancreatic cancer cell growth no matter whether the pyrimidine antimetabolite is effective in inhibiting cancer cell growth.

Example 7

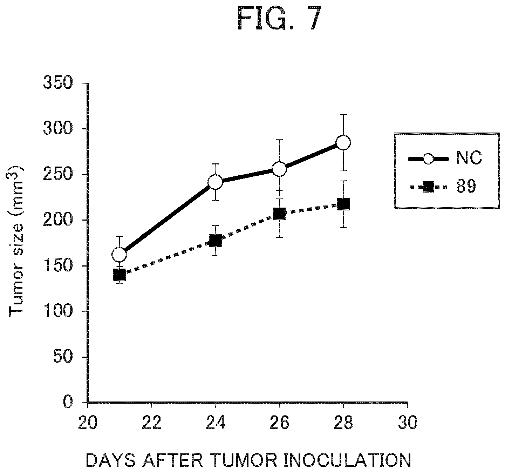

Nude mice (Balb/c-nu/nu, female, 7 weeks old) were inoculated with human liver cancer cells Hep3B ($3 \times 10^6$ cells/mouse), and 21, 24, and 26 days after the average tumor volume reached approximately 150 $mm^3$, gapmer 89 (20 $\mu$g/200 $\mu$L micelles (50% cRGD)/mouse) was administered to the mice via tail vein injection, while 21, 24, 26, and 28 days after the inoculation, the long and short diameters of the tumors were measured with a caliper and used to calculate the tumor volume (tumor size) ($mm^3$). The same test procedure was performed using gapmer NC (negative control (NC)), which targeted a sequence not found in any genome. The results are shown in FIG. 7.

FIG. 7 is a graph showing the results of the pancreatic cancer cell AsPC1 growth inhibition test by the antisense oligonucleotide against MEX3B. In the graph, the error bars indicate the standard errors. The results shown in FIG. 7 indicate that the administration of gapmer 89 reduced the tumor volume as compared to the administration of gapmer NC. This means that gapmer 89 is a substance that reduces expression of the MEX3B gene or the MEX3B protein and is effective in inhibiting liver cancer cell growth.

**Claims**

1.    A prophylactic or therapeutic agent for at least one cancer selected from the group consisting of pancreatic cancer, lung cancer, colorectal cancer, cholangiocarcinoma, and liver cancer, the prophylactic or therapeutic agent comprising a substance that reduces expression of an MEX3B gene or an MEX3B protein or comprising a substance inhibiting an MEX3B protein.

2.    The prophylactic or therapeutic agent according to claim 1, wherein the substance is an antisense oligonucleotide having a sequence complementary to a partial contiguous sequence of the MEX3B gene or having a sequence complementary to a partial contiguous sequence of an expression control region of the MEX3B gene and being capable of inhibiting expression of the MEX3B gene.

3.    The prophylactic or therapeutic agent according to claim 1, wherein the substance is a nucleic acid having RNAi activity or a miRNA, wherein the nucleic acid having RNAi activity or the miRNA comprises a partial contiguous sequence of a coding or untranslated region of an RNA sequence transcribed from the MEX3B gene or comprises a sequence complementary to the partial contiguous sequence and is capable of inhibiting expression of the MEX3B gene.

4.    The prophylactic or therapeutic agent according to any one of claims 1 to 3, wherein the at least one cancer is refractory to an anticancer agent other than the prophylactic or therapeutic agent, the prophylactic or therapeutic agent being for administration to a patient with the cancer refractory to the anticancer agent other than the prophylactic or therapeutic agent.

5. The prophylactic or therapeutic agent according to any one of claims 1 to 4, being for use in combination with an anticancer agent other than the prophylactic or therapeutic agent to form a combination drug.

6. The prophylactic or therapeutic agent according to claim 4 or 5, wherein the anticancer agent other than the prophylactic or therapeutic agent comprises at least one anticancer agent selected from the group consisting of an immune checkpoint inhibitor and a pyrimidine antimetabolite.

7. The prophylactic or therapeutic agent according to any one of claims 1 to 6, wherein the lung cancer is non-small cell lung cancer.

8. A prophylactic or therapeutic agent for at least one cancer selected from the group consisting of pancreatic cancer, lung cancer, colorectal cancer, cholangiocarcinoma, and liver cancer and for use in combination with the prophylactic or therapeutic agent according to any one of claims 1 to 7 to form a combination drug.

9. The prophylactic or therapeutic agent according to claim 8, comprising at least one anticancer agent selected from the group consisting of an immune checkpoint inhibitor and a pyrimidine antimetabolite.

10. A combination drug comprising: the prophylactic or therapeutic agent according to claim 5; and the anticancer agent other than the prophylactic or therapeutic agent.

11. A method for screening for a prophylactic or therapeutic agent for at least one cancer selected from the group consisting of pancreatic cancer, lung cancer, colorectal cancer, cholangiocarcinoma, and liver cancer, the method comprising screening test substances using at least one indicator selected from the group consisting of a reduction in MEX3B gene expression or MEX3B protein expression and a reduction in MEX3B protein function.

12. The method according to claim 11, wherein the prophylactic or therapeutic agent is for use in combination with an additional anticancer agent to form a combination drug.

13. The method according to claim 11 or 12, comprising culturing MEX3B gene-expressing cells in the presence of a test substance and culturing MEX3B gene-expressing cells in the absence of the test substance, wherein the indicator is at least one selected from the group consisting of a reduction in MEX3B gene expression or MEX3B protein expression depending on the presence or absence of the test substance and a reduction in MEX3B protein function depending on the presence or absence of the test substance.

14. The method according to any one of claims 11 to 13, wherein the lung cancer is non-small cell lung cancer.

## FIG. 1A

* p-value < 0.05
(Turkey-Kramer test)

## FIG. 1B

* p-value < 0.05
(Turkey-Kramer test)

# FIG. 2

# FIG. 3A

# FIG. 3B

# FIG. 4

HUCCT1

* p-value < 0.01

# FIG. 5A

CT26.WT cell
(1 x 10⁵ cell/100 ul)injection
(SC)

8 μg Gapmer with in
vivo jetPEI in 80 μl
solution/mice

8 week ♂
Balb/c

6 days

IT   IT   IT      IT      IT   Sampling

6    8    10      14      17      20    day

IP(##)  IP(#)  IP(#)

SC : subcutaneous
IT : Intra-tumoral
IP : Intraperitoneal

PD-L1 antibody
100(#) or 200(##) μg/mice

# FIG. 5B

α-PD-L1+LNA-NC

Tumor Volume (mm³)

DAYS AFTER TUMOR INOCULATION

↓ INTRATUMORAL INJECTION OF GAPMER

⇣ INTRAPERITONEAL INJECTION OF
ANTI-PD-L1 ANTIBODY

# FIG. 5C

α-PD-L1+LNA-89

DAYS AFTER TUMOR INOCULATION

↓ INTRATUMORAL INJECTION OF GAPMER

↓ INTRAPERITONEAL INJECTION OF ANTI-PD-L1 ANTIBODY

# FIG. 5D

LNA-NC   LNA-89

+α-PDL1

# FIG. 6A

## FIG. 6B

## FIG. 7

## INTERNATIONAL SEARCH REPORT

| International application No. |
| --- |
| PCT/JP2021/017019 |

### A. CLASSIFICATION OF SUBJECT MATTER

Int. Cl. A61K39/395(2006.01)i, A61K45/00(2006.01)i, A61K48/00(2006.01)i, A61P1/00(2006.01)i, A61P1/18(2006.01)i, A61P11/00(2006.01)i, A61P35/00(2006.01)i, A61P43/00(2006.01)i, C12N15/113(2010.01)i, C12Q1/6886(2018.01)i, G01N33/15(2006.01)i, G01N33/50(2006.01)i, A61K31/7068(2006.01)i, A61K31/7088(2006.01)i, A61K31/7105(2006.01)i, A61K31/713(2006.01)i
FI: A61K45/00, C12Q1/6886 Z, A61P35/00, A61K31/7088, A61K31/713, A61K31/7105, A61P43/00 121, A61P1/18, A61P11/00, A61P1/00, A61K48/00, A61K39/395 N, A61K31/7068, G01N33/15 Z, G01N33/50 Z, C12N15/113 Z ZNA

According to International Patent Classification (IPC) or to both national classification and IPC

### B. FIELDS SEARCHED

Minimum documentation searched (classification system followed by classification symbols)
Int. Cl. A61K39/395, A61K45/00, A61K48/00, A61P1/00, A61P1/18, A61P11/00, A61P35/00, A61P43/00, C12N15/113, C12Q1/6886, G01N33/15, G01N33/50, A61K31/7068, A61K31/7088, A61K31/7105, A61K31/713

Documentation searched other than minimum documentation to the extent that such documents are included in the fields searched
Published examined utility model applications of Japan     1922-1996
Published unexamined utility model applications of Japan   1971-2021
Registered utility model specifications of Japan           1996-2021
Published registered utility model applications of Japan   1994-2021

Electronic data base consulted during the international search (name of data base and, where practicable, search terms used)
JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| A | WO 2011/036118 A1 (NOVARTIS FORSCHUNGSSTIFTUNG, ZWEIGNLEDELASSUNG FRIEDRICH MIESCHER INSTITUTE FOR BIOMEDICAL RESEARCH) 31 March 2011, entire text | 1-14 |
| A | XUE, M. et al. HOTAIR induces the ubiquitination of Runx3 by interacting with Mex3b and enhances the invasion of gastric cancer cells Gastric Cancer, 2018, vol. 21, pp. 756-764, entire text | 1-14 |
| A | @HUANG, L. et al. The RNA-binding Protein MEX3B Mediates Resistance to Cancer Immunotherapy by Downregulating HLA-A Expression, Clinical Cancer Research, 2018, vol. 24, no. 14, pp. 3366-3376, entire text | 1-14 |

☒ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 09.06.2021 | 29.06.2021 |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (January 2015)

**INTERNATIONAL SEARCH REPORT**

| International application No. |
| --- |
| PCT/JP2021/017019 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | WO 2018/008750 A1 (TAK-CIRCULATOR CO., LTD.) 11 January 2018, entire text | 1-14 |
| A | JP 2018-11593 A (TAK-CIRCULATOR CO., LTD.) 25 January 2018, entire text | 1-14 |
| A | JIANG, H. et al. Knockdown of hMex-3A by small RNA interference suppresses cell proliferation and migration in human gastric cancer cells. Molecular Medicine Reports, 2012, vol. 6, pp. 575-580, entire text | 1-14 |
| P, A | JASINSKI-BERGNER, S. et al. The Role of the RNA-Binding Protein Family MEX-3 in Tumorigenesis. International Journal of Molecular Sciences, July 2020, vol. 21, 5209, pp. 1-14, entire text | 1-14 |

Form PCT/ISA/210 (continuation of second sheet) (January 2015)

**INTERNATIONAL SEARCH REPORT**

Information on patent family members

| International application No. |
| --- |
| PCT/JP2021/017019 |

| Patent Documents referred to in the Report | Publication Date | Patent Family | Publication Date |
| --- | --- | --- | --- |
| WO 2011/036118 A1 | 31.03.2011 | US 2012/0244170 A1<br>EP 2480573 A1 | |
| WO 2018/008750 A1 | 11.01.2018 | EP 3483283 A1<br>entire document<br>CN 109526229 A | |
| JP 2018-11593 A | 25.01.2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4409430 B
- WO 2018008750 A1
- JP 4429269 B

**Non-patent literature cited in the description**

- *Oncogene,* September 2018, vol. 37 (38), 5233-5247
- *Cell Rep.,* 30 August 2016, vol. 16 (9), 2456-71
- *Oncogene.,* September 2018, vol. 37 (38), 5233-5247
- **MIYATA K. et al.** *React. Funct. Polym.,* 2011, vol. 71, 227-234
- **MIYATA K.** *Drug Discov. Ther.,* 2016, vol. 10, 236-247
- **MORRISON, S. L. et al.** *Proc. Natl. Acad. Sci. USA,* 1984, vol. 81, 6851-6855
- **HIROSHI NOGUCHI.** *Igaku no ayumi,* 1993, vol. 167, 457-462